(19) Europäisches Patentamt
European Patent Office
Office européen des brevets

(11) EP 4 700 895 A1

(12) EUROPEAN PATENT APPLICATION
published in accordance with Art. 153(4) EPC

(43) Date of publication:
**25.02.2026 Bulletin 2026/09**

(21) Application number: **24908110.0**

(22) Date of filing: **19.12.2024**

(51) International Patent Classification (IPC):
***H01M 10/0567*** *(2010.01)* ***H01M 10/0525*** *(2010.01)*
***H01M 4/525*** *(2010.01)* ***H01M 4/505*** *(2010.01)*

(52) Cooperative Patent Classification (CPC):
**C07D 411/12; C07D 411/08; H01M 10/0525; H01M 10/0567; H01M 10/0568; H01M 10/0569;**
H01M 2004/028; H01M 2300/0025; Y02E 60/10

(86) International application number:
**PCT/KR2024/020701**

(87) International publication number:
**WO 2025/135827 (26.06.2025 Gazette 2025/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **20.12.2023 KR 20230187752**

(71) Applicant: **LG Energy Solution, Ltd.**
**Seoul 07335 (KR)**

(72) Inventors:
- **JI, Su Hyeon**
**Daejeon 34122 (KR)**
- **LEE, Chul Haeng**
**Daejeon 34122 (KR)**
- **LEE, Jung Min**
**Daejeon 34122 (KR)**
- **YEOM, Chul Eun**
**Daejeon 34122 (KR)**
- **KO, Jin Hyuck**
**Daejeon 34122 (KR)**

(74) Representative: **Hoffmann Eitle**
**Patent- und Rechtsanwälte PartmbB**
**Arabellastraße 30**
**81925 München (DE)**

# (54) NON-AQUEOUS ELECTROLYTE AND LITHIUM SECONDARY BATTERY COMPRISING SAME

(57) The present invention provides a non-aqueous electrolyte comprising: a lithium salt; an organic solvent; and an additive, wherein the additive comprises a compound represented by a specific chemical formula. The non-aqueous electrolyte forms a stable film on a positive electrode and a negative electrode, thereby improving long-term durability and lifespan performance of a lithium secondary battery.



Processed by Luminess, 75001 PARIS (FR)

## Description

### TECHNICAL FIELD

The present invention relates to a non-aqueous electrolyte and a lithium secondary battery including the same.

### BACKGROUNDART

Recently, as the application areas of lithium secondary batteries have rapidly expanded to include not only power supply for electronic devices such as electric, electronic, communication, and computers, but also power storage for large-area devices such as automobiles and power storage devices, the demand for high-capacity, high-output, and high-stability secondary batteries is increasing.

The lithium secondary batteries are generally constituted with a positive electrode including a positive electrode active material, a negative electrode including a negative electrode active material, an electrolyte that serves as a medium for transferring lithium ions, and a separator. In this case, the negative electrode active material may be a carbon-based active material or a silicon-based active material. In addition, the positive electrode active material may be lithium transition metal oxides such as lithium cobalt oxide ($LiCoO_2$), lithium nickel oxide ($LiNiO_2$), and lithium nickel-cobalt-manganese composite oxide.

Meanwhile, in recent years, lithium transition metal oxides containing more than 80 mol% of nickel, as compared to transition metals, have been studied to increase the energy density of the cathode. However, in the case of such high-nickel lithium transition metal composite oxides, the high nickel content leads to deterioration in the thermal stability of the cathode, which poses a problem.

Alternatively, in consideration of the thermal stability issues that arise when increasing the nickel content, the use of lithium transition metal composite oxides with an appropriately reduced nickel content is also being considered. However, when the nickel content is reduced, a higher operating voltage is required to achieve the desired energy density. Under such high-voltage operation, issues may occur such as side reactions between the cathode and electrolyte, reduced high-temperature durability, and increased resistance.

In order to achieve high energy density in such cathodes and lithium secondary batteries containing them, it is necessary to resolve problems such as thermal instability, high-temperature durability degradation, and electrolyte side reactions.

### DISCLOSURE OF THE INVENTION

### TECHNICAL PROBLEM

One object of the present invention is to solve the aforementioned problems and to provide a non-aqueous electrolyte capable of improving high-temperature durability of both a positive electrode and a negative electrode, preventing side reactions with the electrolyte, and particularly enhancing long-term durability, cycle life performance, and storage performance.

Another object of the present invention is to provide a lithium secondary battery comprising the above non-aqueous electrolyte.

### MEANS FOR SOLVING THE PROBLEM

[1] The present invention provides a non-aqueous electrolyte including: a lithium salt; an organic solvent; and an additive, wherein the additive contains a compound represented by Formula 1:

(Formula 1)

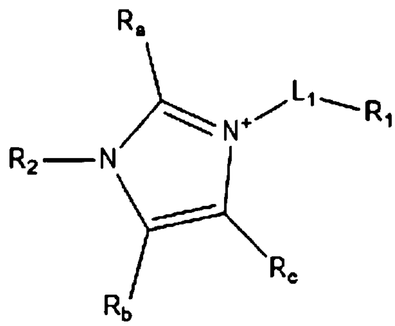

In Formula 1, $R_1$ is a substituent represented by Formula 2 below, $L_1$ is a direct bond or a substituted or unsubstituted alkylene group having 1 to 6 carbon atoms, $R_2$ is hydrogen or a substituent represented by Formula 3 below, and $R_a$, $R_b$, and $R_c$ are each independently hydrogen, an alkyl group having 1 to 3 carbon atoms, or -CN.

(Formula 2)

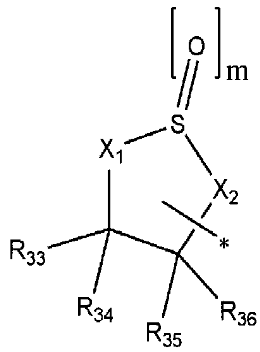

In Formula 2, m is 1 or 2, $X_1$ and $X_2$ are each independently -O- or $-C(R_{31})(R_{32})-$, provided that at least one of $X_1$ and $X_2$ is -O-, $R_{31}$ to $R_{36}$ are each independently hydrogen, an alkyl group having 1 to 6 carbon atoms, $-C(=O)-R_4$, or $-R_5-O-C(=O)-R_6$, $R_4$ and $R_6$ are each independently a substituted or unsubstituted alkyl group having 1 to 6 carbon atoms, an alkenyl group having 2 to 20 carbon atoms, an alkynyl group having 2 to 20 carbon atoms, or a substituted or unsubstituted aryl group having 6 to 20 carbon atoms, $R_5$ is a substituted or unsubstituted alkylene group having 1 to 6 carbon atoms, each of substituents of $L_1$, $R_4$, $R_5$, and $R_6$ are each independently at least one selected from the group consisting of deuterium, -F, -Cl, -Br, -I, -CN, $-NO_2$, and $-SO_3$, and * is a bonding site located at one of $R_{31}$, $R_{32}$, $R_{33}$, $R_{34}$, $R_{35}$, and $R_{36}$.

(Formula 3)

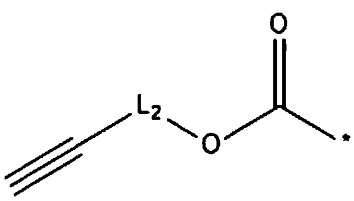

In Formula 3, * is a bonding site, and $L_2$ is an alkylene group having 1 to 3 carbon atoms.

[2] The present invention provides the non-aqueous electrolyte according to the above [1], wherein the compound represented by Formula 1 includes at least one of compounds represented by Formulas 1-1 and 1-2:

(Formula 1-1)

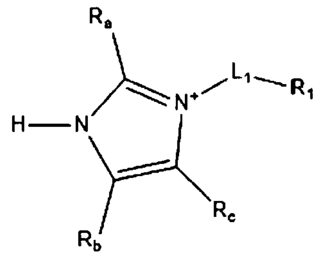

(Formula 1-2)

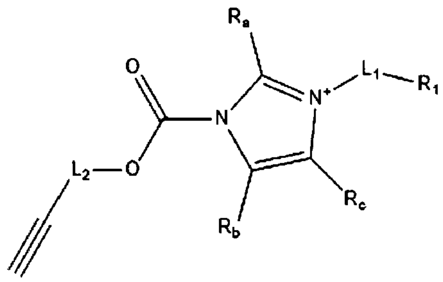

wherein $R_a$, $R_b$, $R_c$, $R_1$, $L_1$, and $L_2$ are as defined in Formula 1.

[3] The present invention provides the non-aqueous electrolyte according to at least one of the above [1] to [2],wherein $L_1$ is a methylene group.

[4] The present invention provides the non-aqueous electrolyte according to at least one of the above [1] to [3], wherein the substituent represented by Formula 2 is any one selected from substituents CS-1 to CS-15:

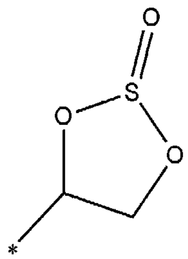

CS-1

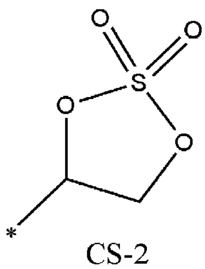

CS-2

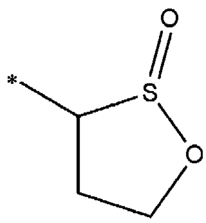

CS-3

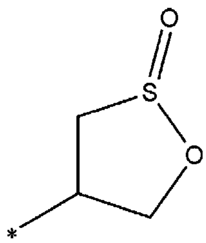

CS-4

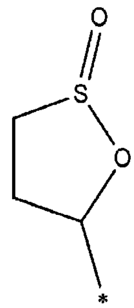

CS-5

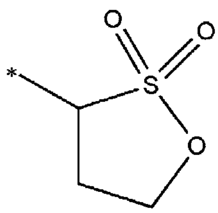

CS-6

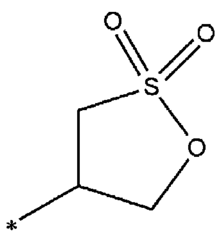

CS-7

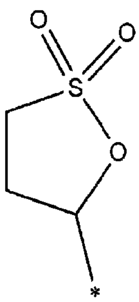

CS-8

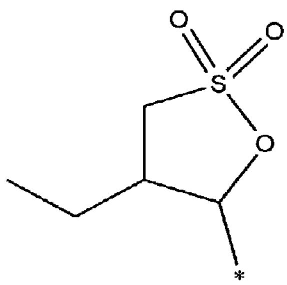

CS-9

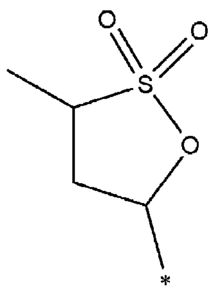

CS-10

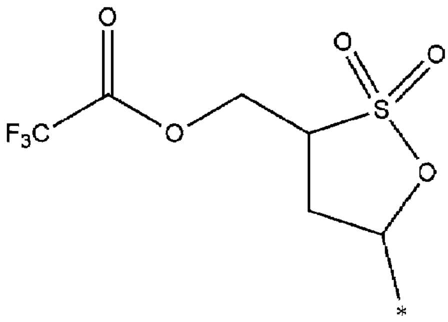

CS-11

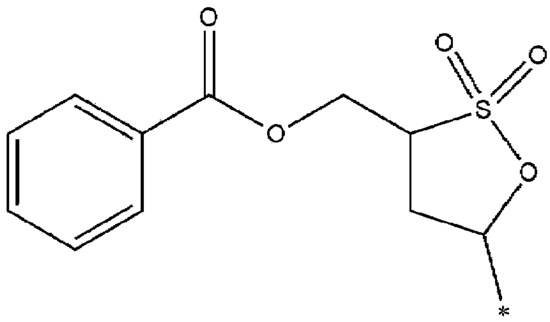

CS-12

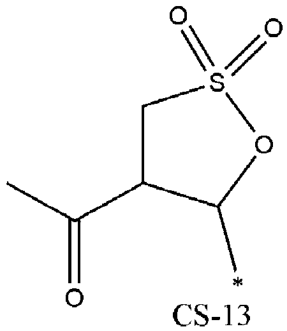

CS-13

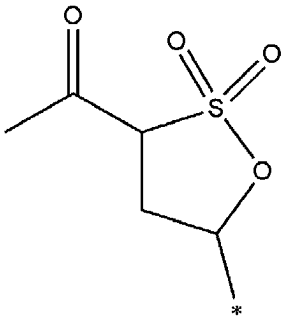

CS-14

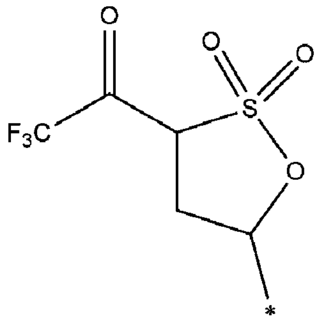

CS-15

[5] The present invention provides the non-aqueous electrolyte according to at least one of the above [1] to [4],wherein the substituent represented by Formula 2 is one selected from the group consisting of CS-1, CS-2, CS-5, CS-8, CS-10, and CS-11.

[6] The present invention provides the non-aqueous electrolyte according to at least one of the above [1] to [5], wherein $L_2$ is a methylene group.

[7] The present invention provides the non-aqueous electrolyte according to at least one of the above [1] to [6], wherein the compound represented by Formula 1 includes at least one of compounds represented by Formulas 1-A and 1-B:

(Formula 1-A)

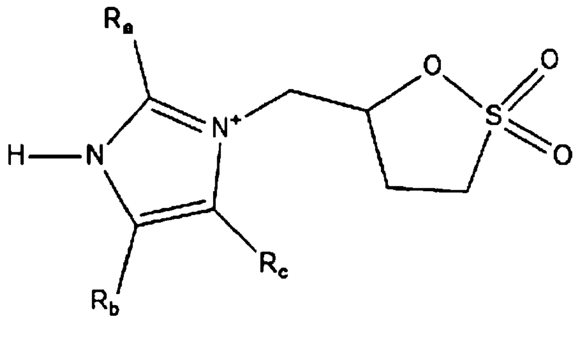

(Formula 1-B)

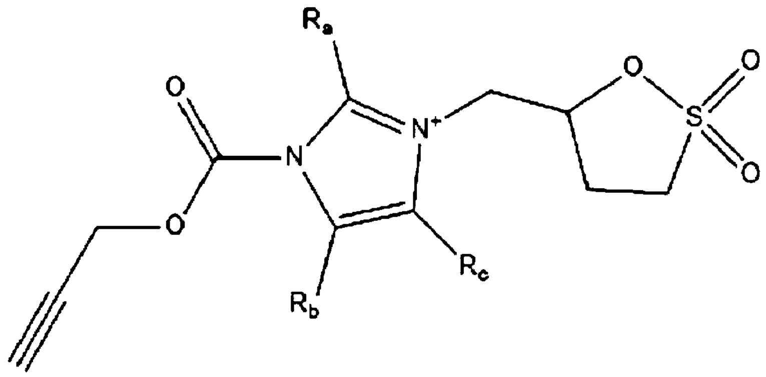

wherein $R_a$, $R_b$, and $R_c$ are as defined in Formula 1.

[8] The present invention provides the non-aqueous electrolyte according to at least one of the above [1] to [7], wherein the compound represented by Formula 1 includes at least one of compounds represented by Formulas 1-a and 1-b:

(Formula 1-a)

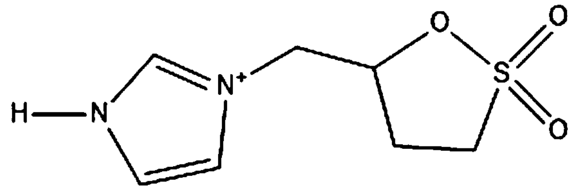

(Formula 1-b)

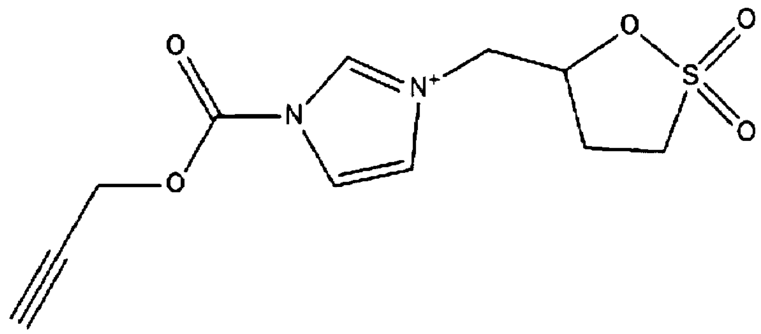

[9] The present invention provides the non-aqueous electrolyte according to at least one of the above [1] to [8], wherein the additive further includes a compound represented by Formula 4:

(Formula 4)

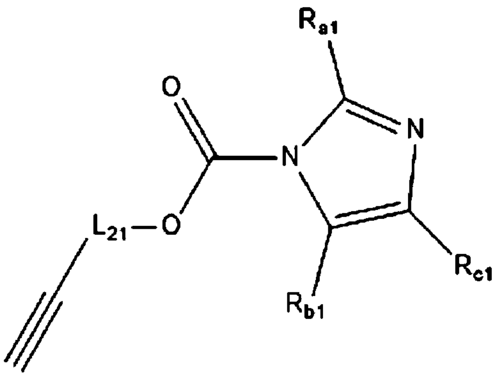

wherein, $R_{a1}$, $R_{b1}$ and $R_{c1}$ are each independently hydrogen, an alkyl group having 1 to 3 carbon atoms, or -CN, and $L_{21}$ is an alkylene group having 1 to 3 carbon atoms.

[10] The present invention provides the non-aqueous electrolyte according to at least one of the above [1] to [9], wherein the additive further includes a compound represented by Formula 5:

(Formula 5)

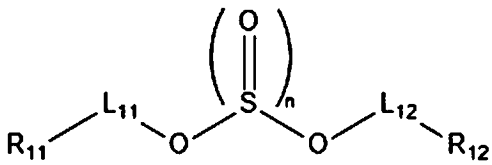

wherein, n is 1 or 2, $L_{11}$ and $L_{12}$ are each independently a direct bond or a substituted or unsubstituted alkylene group having 1 to 6 carbon atoms, and $R_{11}$ and $R_{12}$ are each independently a substituent represented by Formula 6:

(Formula 6)

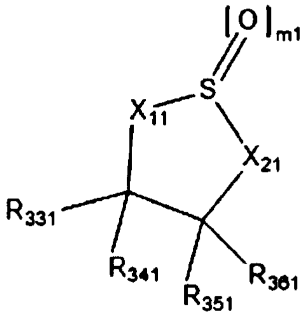

wherein, m1 is 1 or 2, $X_{11}$ and $X_{21}$ are each independently -O- or -C($R_{311}$)($R_{321}$)-, provided that at least one of $X_{11}$ and $X_{21}$ is -O-, $R_{311}$, $R_{321}$, $R_{331}$, $R_{341}$, $R_{351}$, and $R_{361}$ are each independently hydrogen, an alkyl group having 1 to 6 carbon atoms, -C(=O)-$R_{41}$, or -$R_{51}$-O-C(=O)-$R_{61}$, $R_{41}$ and $R_{61}$ are each independently a substituted or unsubstituted alkyl group having 1 to 6 carbon atoms, an alkenyl group having 2 to 20 carbon atoms, an alkynyl group having 2 to 20 carbon atoms, or substituted or unsubstituted aryl group having 6 to 20 carbon atoms, $R_{51}$ is a substituted or unsubstituted alkylene group having 1 to 6 carbon atoms, each of substituents of $L_{11}$, $L_{21}$, $R_{41}$, $R_{51}$, and $R_{61}$ are each independently at least one selected from the group consisting of deuterium, -F, -Cl, -Br, -I, -CN, $-NO_2$, and $-SO_3$, * is a bonding site located at one of $R_{311}$, $R_{321}$, $R_{331}$, $R_{341}$, $R_{351}$, and $R_{361}$, when $L_{11}$ and $L_{21}$ are both direct bonds, $R_{11}$ and $R_{21}$ are not CS1-7 below at the same time, and when $L_{11}$ and $L_{21}$ are both methylene groups and n is 2, $R_{11}$ and $R_{21}$ are not CS1-2 below at the same time:

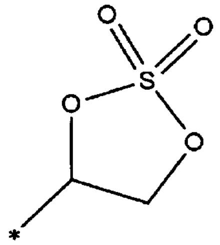

CS1-2

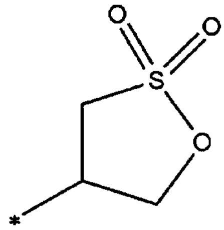

CS1-7

[11] The present invention provides the non-aqueous electrolyte according to at least one of the above [1] to [10], wherein the substituent represented by Formula 6 is any one selected from the group consisting of CS1-1 to CS1-15:

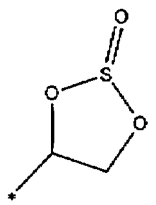

CS1-1

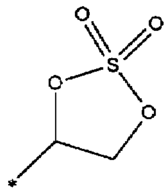

CS1-2

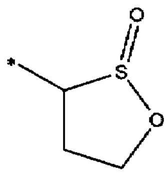

CS1-3

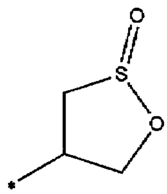

CS1-4

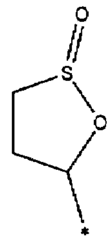

CS1-5

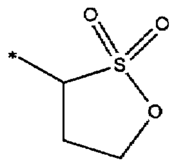

CS1-6

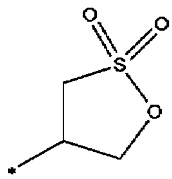

CS1-7

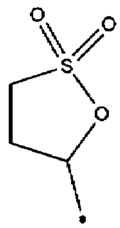

CS1-8

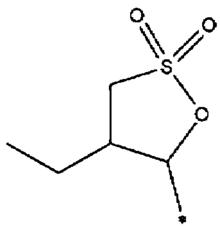

CS1-9

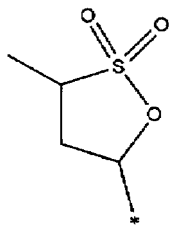

CS1-10

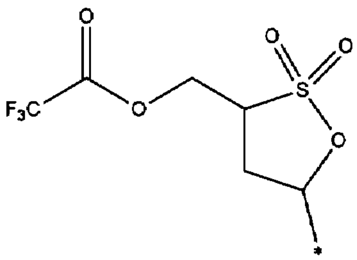

CS1-11

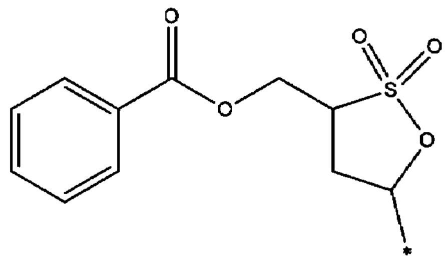

CS1-12

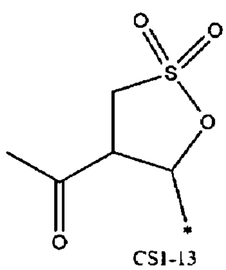

CS1-13

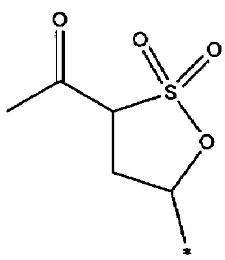

CS1-14

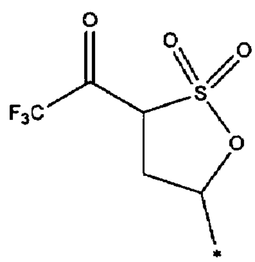

CS1-15

[12] The present invention provides the non-aqueous electrolyte according to at least one of the above [1] to [11], wherein the compound represented by Formula 5 includes at least one compound selected from the group consisting of compounds A to Q:

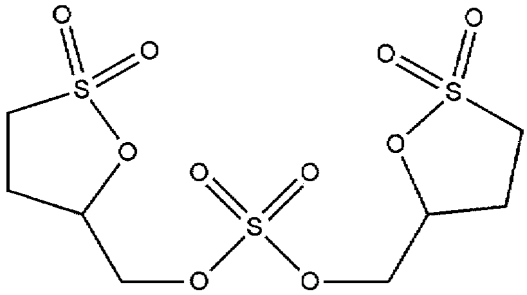

A

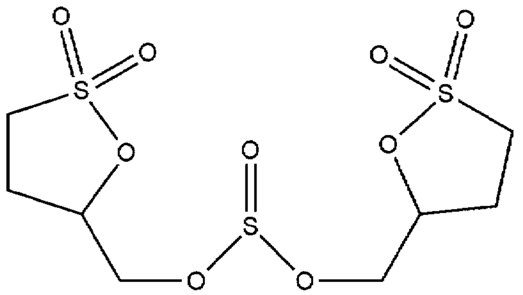

B

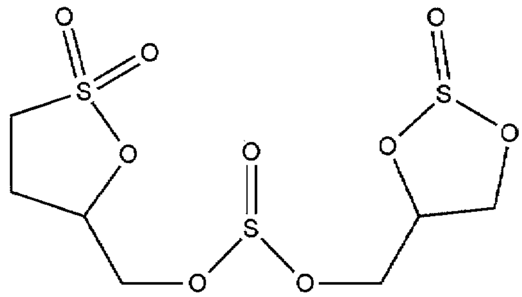

C

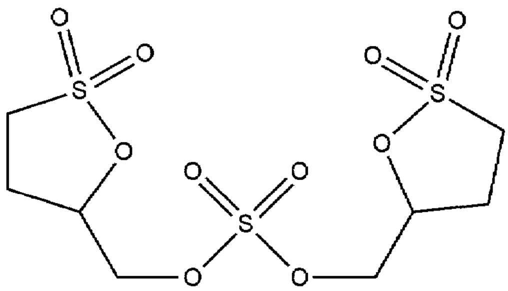

D

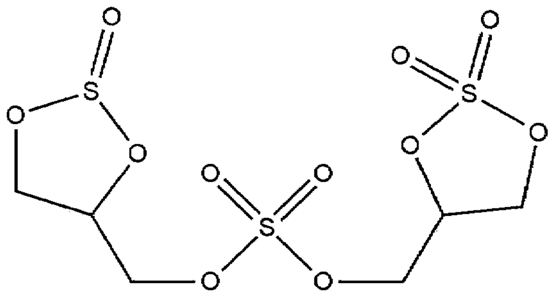

E

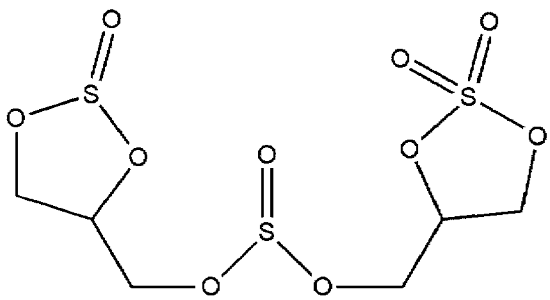

F

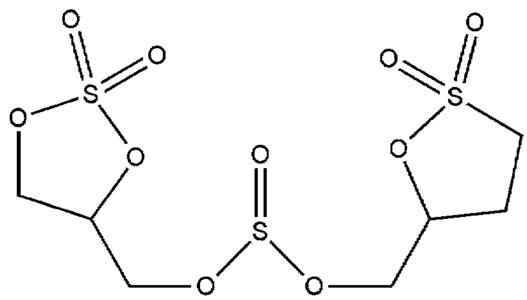

G

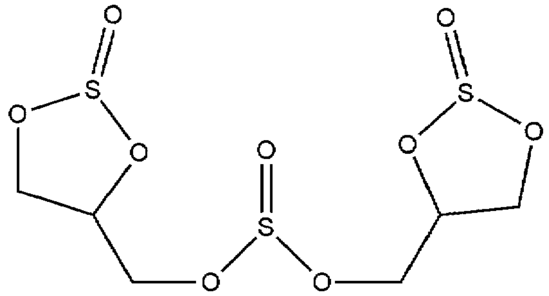

H

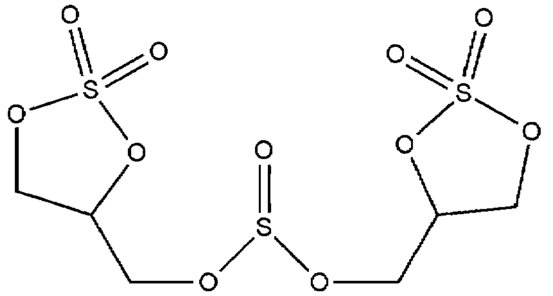

I

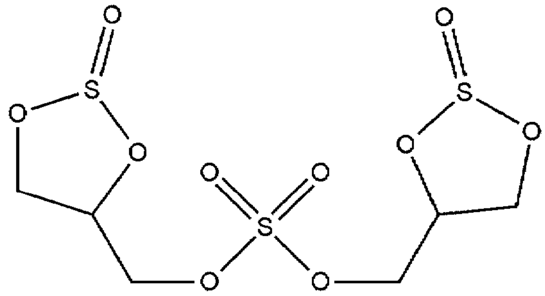

J

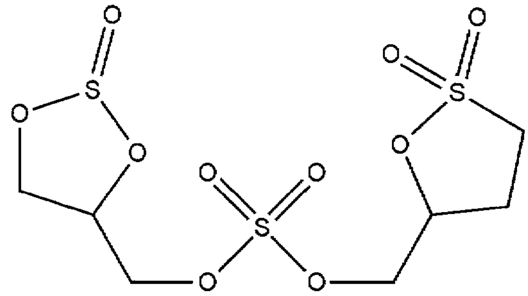

K

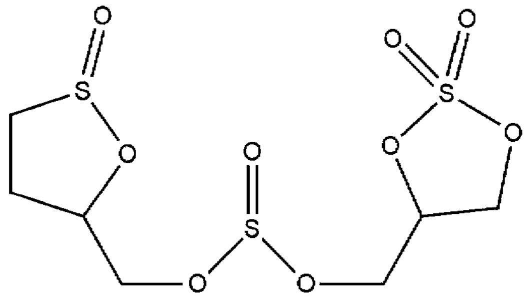

L

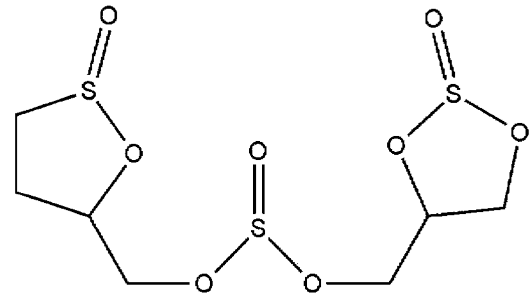

M

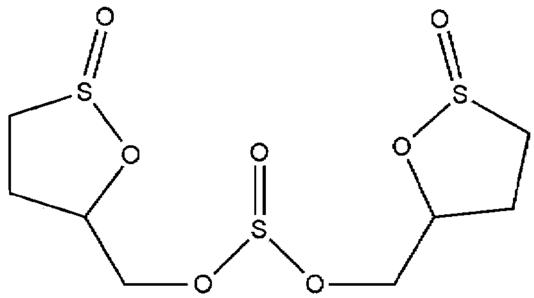

N

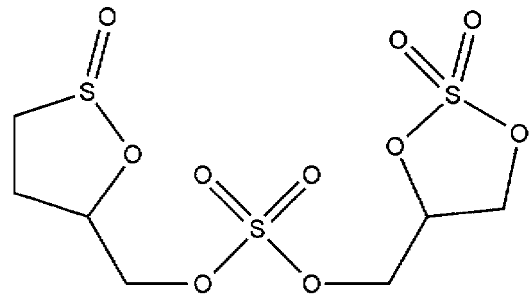

O

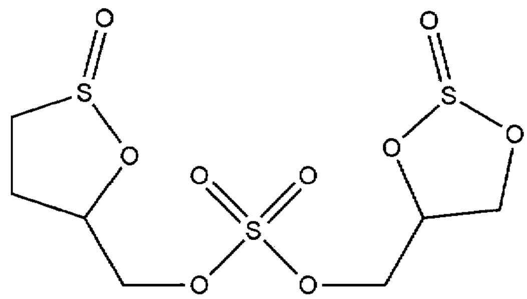

P

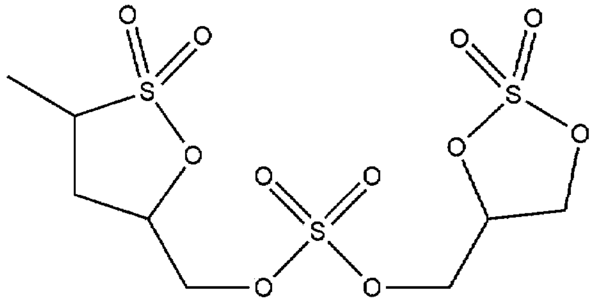

Q

[13] The present invention provides the non-aqueous electrolyte according to at least one of the above [1] to [12], wherein the compound represented by Formula 1 is included in an amount of 0.01 wt% to 10 wt% based on the total weight of the non-aqueous electrolyte.

[14] The present invention provides the non-aqueous electrolyte according to at least one of the above [1] to [13], wherein the lithium salt incudes at least one selected from the group consisting of LiCl, LiBr, LiI, $LiBF_4$, $LiClO_4$, $LiAlO_4$, $LiAlCl_4$, $LiPF_6$, $LiSbF_6$, $LiAsF_6$, $LiB_{10}Cl_{10}$, LiBOB ($LiB(C_2O_4)_2$), $LiCF_3SO_3$, LiFSI ($LiN(SO_2F)_2$), $LiCH_3SO_3$, $LiCF_3CO_2$, $LiCH_3CO_2$, and LiBETI ($LiN(SO_2CF_2CF_3)_2$).

[15] The present invention provides a lithium secondary battery comprising: a positive electrode; a negative electrode disposed in an opposite side to the positive electrode; a separator interposed between the positive electrode and the negative electrode; and the non-aqueous electrolyte according to at least one of the above [1] to [14].

[16] The present invention provides the lithium secondary battery according to [15], wherein the positive electrode includes a positive electrode active material, and the positive electrode active material includes a lithium transition metal oxide represented by Formula P-1:

(Formula P-1) $Li_{1+x}[Ni_aCo_bMn_cM^1{}_d]O_{2+w}$

wherein, x, a, b, c, d, and w satisfy $0 \le x \le 0.5$, $a+b+c+d = 1$, $0.5 \le a \le 0.7$, $0 \le b \le 0.15$, $c=1-a-b-d$, $0 \le d \le 0.1$, $0 \le b/a \le 0.2$, $1 \le a/c \le 3$, $0 \le w \le 1$, respectively, and $M^1$ is at least one selected from W, Cu, Fe, V, Cr, Ti, Zr, Zn, Al, In, Ta, Y, La, Sr, Ga, Sc, Gd, Sm, Ca, Ce, Nb, Mg, B, and Mo.

## EFFECT OF THE INVENTION

The non-aqueous electrolyte of the present invention includes, as an additive, a compound of a specific chemical formula having an imidazolium cation structure substituted with a cyclic sulfur oxide. When the compound is used as an additive, it may continuously contribute to the formation of films on the positive and negative electrodes even during long-term charge and discharge, thereby expecting improving long-term life performance, high-temperature durability, and thermal stability.

Thus, a lithium secondary battery including the non-aqueous electrolyte described above may have excellent life performance and high-temperature storage characteristics. Particularly, when applied to a lithium secondary battery requiring high energy density and high-voltage operation, significantly excellent life performance and high-temperature storage performance improvement may be expected.

## MODE FOR CARRYING OUR THE INVENTION

Before describing the present invention, it should be noted that the terms or words used in this specification and claims should not be interpreted as limited to their usual or dictionary meanings, and should be interpreted as meanings and concepts that conform to the technical idea of the present invention based on the principle that the inventors can appropriately define the concept of the term in order to explain their own invention in the best manner.

The terms used herein are only used to describe embodiments, and are not intended to limit the present invention. Singular expressions include plural expressions unless the context clearly indicates otherwise.

As used herein, it should be understood that the terms "comprise,""include," or "have" are intended to specify the presence of a feature, number, step, component, or combination thereof, but do not exclude in advance the possibility of the presence or addition of one or more other features, numbers, steps, components, or combinations thereof.

As used herein, "%" means % by weight unless otherwise explicitly indicated.

Before explaining the present invention, in the description of "carbon number a to b" in the specification, "a" and

"b" mean the number of carbon atoms included in a functional group. That is, the functional group may include "a" to "b" carbon atoms.

Also, as used herein, "substituted" means that at least one hydrogen bonded to a carbon is substituted with an element other than hydrogen, for example, an alkyl group having 1 to 5 carbon atoms or a fluorine element, unless otherwise defined.

In this specification, the average particle diameter (D50) may be defined as a particle diameter corresponding to 50% of the volume accumulation amount in the particle diameter distribution curve of the particles. The average particle diameter (D50) may be measured using, for example, a laser diffraction method. The laser diffraction method is generally capable of measuring particle sizes from the submicron region down to a few millimeters and may obtain results with high reproducibility and high resolution.

Hereinafter, the present invention will be described in more detail.

## Non-aqueous Electrolyte

The present invention provides a non-aqueous electrolyte, specifically a non-aqueous electrolyte for a lithium secondary battery.

More specifically, the non-aqueous electrolyte according to the present invention includes a lithium salt; an organic solvent; and an additive, in which the additive contains a compound represented by Formula 1:

(Formula 1)

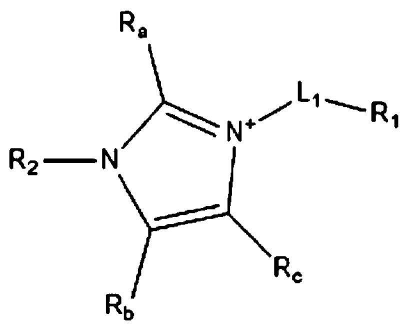

In Formula 1, $R_1$ is a substituent represented by Formula 2 below, $L_1$ is a direct bond or a substituted or unsubstituted alkylene group having 1 to 6 carbon atoms, $R_2$ is hydrogen or a substituent represented by Formula 3 below, and $R_a$, $R_b$, and $R_c$ are each independently hydrogen, an alkyl group having 1 to 3 carbon atoms, or -CN.

(Formula 2)

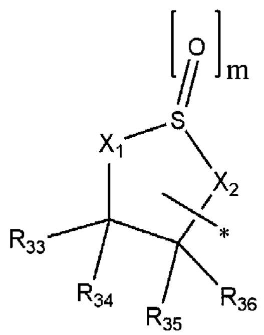

In Formula 2, m is 1 or 2, $X_1$ and $X_2$ are each independently -O- or -C($R_{31}$)($R_{32}$)-, provided that at least one of $X_1$ and $X_2$ is -O-, $R_{31}$ to $R_{36}$ are each independently hydrogen, an alkyl group having 1 to 6 carbon atoms, -C(=O)-$R_4$, or -$R_5$-O-C(=O)-$R_6$, $R_4$ and $R_6$ are each independently a substituted or unsubstituted alkyl group having 1 to 6 carbon atoms, an alkenyl group having 2 to 20 carbon atoms, an alkynyl group having 2 to 20 carbon atoms, or a substituted or unsubstituted aryl group having 6 to 20 carbon atoms, $R_5$ is a substituted or unsubstituted alkylene group having 1 to 6 carbon atoms, each of substituents of $L_1$, $R_4$, $R_5$, and $R_6$ are each independently at least one selected fromthe group consisting of deuterium, -F, -Cl, -Br, -I, -CN, $-NO_2$, and $-SO_3$, and * is a bonding site located at one of $R_{31}$, $R_{32}$, $R_{33}$, $R_{34}$, $R_{35}$, and $R_{36}$.

(Formula 3)

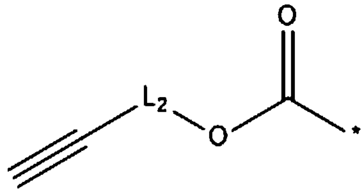

In Formula 3, * is a bonding site, and $L_2$ is an alkylene group having 1 to 3 carbon atoms.

The non-aqueous electrolyte of the present invention includes, as an additive, a compound of a specific chemical formula having an imidazolium cation structure substituted with a cyclic sulfur oxide. When the compound is used as an additive, it may continuously contribute to the formation of films on the positive and negative electrodes even during long-term charge and discharge, thereby expecting improving long-term life performance, high-temperature durability, and thermal stability. Accordingly, a lithium secondary battery including the non-aqueous electrolyte described above may have excellent life performance and high-temperature storage characteristics. When applied to a lithium secondary battery requiring high energy density and high-voltage operation, significantly excellent life performance and high-temperature storage performance improvement may be expected.

(1) Lithium Salt

First, a lithium salt is described as follows.

In the non-aqueous electrolyte for a lithium secondary battery according to one embodiment of the present invention, the lithium salt may be any of those commonly used in electrolytes for lithium secondary batteries without limitation, and may be, for example, $Li^+$ as a cation, and, as an anion, at least one selected from the group consisting of $F^-$, $Cl^-$, $Br^-$, $I^-$, $NO_3^-$, $N(CN)_2$, $BF_4^-$, $ClO_4^-$, $AlO_4^-$, $AlCl_4^-$, $PF_6^-$, $SbF_6^-$, $AsF_6^-$, $B_{10}C1_{10}^-$, $BF_2C_2O_4^-$, $BC_4O_8^-$, $PF_4C_2O_4^-$, $PF_2C_4O_8^-$, $(CF_3)_2PF_4^-$, $(CF_3)_3PF_3^-$, $(CF_3)_4PF_2^-$, $(CF_3)_5PF^-$, $(CF_3)_6P^-$, $CF_3SO_3^-$, $C_4F_9SO_3^-$, $CF_3CF_2SO_3^-$, $(CF_3SO_2)_2N^-$, $(FSO_2)_2N^-$, $CF_3CF_2(CF_3)_2CO^-$, $(CF_3SO_2)_2CH^-$, $CH_3SO_3^-$, $CF_3(CF_2)_7SO_3^-$, $CF_3CO_2^-$, $CH_3CO_2^-$, $SCN^-$, and $(CF_3CF_2SO_2)_2N^-$. Specifically, the lithium salt may be at least one selected from the group consisting of LiCl, LiBr, LiI, $LiBF_4$, $LiClO_4$, $LiAlO_4$, $LiAlCl_4$, $LiPF_6$, $LiSbF_6$, $LiAsF_6$, $LiB_{10}Cl_{10}$, LiBOB ($LiB(C_2O_4)_2$), $LiCF_3SO_3$, LiTFSI ($LiN(SO_2CF_3)_2$), LiFSI ($LiN(SO_2F)_2$), $LiCH_3SO_3$, $LiCF_3CO_2$, $LiCH_3CO_2$, and LiBETI ($LiN(SO_2CF_2CF_3)_2$). Specifically, the lithium salt may be one selected from the group consisting of $LiBF_4$, $LiClO_4$, $LiPF_6$, LiBOB ($LiB(C_2O_4)_2$), $LiCF_3SO_3$, LiTFSI ($LiN(SO_2CF_3)_2$), LiFSI ($LiN(SO_2F)_2$), and LiBETI ($LiN(SO_2CF_2CF_3)_2$) either alone or in mixture of two or more thereof, more specifically, may be $LiPF_6$.

The lithium salt may be appropriately changed within a normally usable range, but may be included in the electrolyte at a concentration of 0.8 M to 3.0 M, specifically 1.0 M to 3.0 M, in order to obtain the effect of forming a film for suppressing corrosion on the electrode surface. In this case, the unit "M" may refer to a molar concentration, specifically, which means "mol/L."

When the concentration of the lithium salt satisfies the above range, the viscosity of the non-aqueous electrolyte may be controlled to implement optimal impregnation property, and the mobility of lithium ions may be improved, thereby obtaining the effect of improving the capacity characteristics and cycle characteristics of the lithium secondary battery.

(2) Organic Solvent

The organic solvent is a non-aqueous solvent commonly used in lithium secondary batteries, and is not particularly limited as long as decomposition due to oxidation reaction during the charge and discharge process of the secondary battery is capable of being minimized.

Specifically, the organic solvent may include at least one selected from the group consisting of a cyclic carbonate-based organic solvent, a linear carbonate-based organic solvent, a linear ester-based organic solvent, and a cyclic ester-based organic solvent.

Specifically, the organic solvent may include a cyclic carbonate-based organic solvent, a linear carbonate-based organic solvent, or a mixture thereof.

The cyclic carbonate-based organic solvent is an organic solvent having a high viscosity and a high dielectric constant, and capable of well dissociating a lithium salt in the electrolyte. Specifically, the cyclic carbonate-based organic solvent may include at least one organic solvent selected from ethylene carbonate (EC), fluoroethylene carbonate (FEC), propylene carbonate (PC), 1,2-butylene carbonate, 2,3-butylene carbonate, 1,2-pentylene carbonate, 2,3-pentylene carbonate, and vinylene carbonate, and more specifically, may include at least one selected from ethylene carbonate (EC) and fluoroethylene carbonate (FEC).

In addition, the linear carbonate-based organic solvent is an organic solvent having a low viscosity and a low

dielectric constant. Specifically, the linear carbonate-based organic solvent may include at least one selected fromthe group consisting of dimethyl carbonate (DMC), diethyl carbonate (DEC), and dipropyl carbonate, ethylmethyl carbonate (EMC), methylpropyl carbonate, and ethylpropyl carbonate, more specifically, may include at least one selected from the group consisting of ethylmethyl carbonate (EMC) and diethyl carbonate (DEC).

The organic solvent may be a cyclic carbonate-based organic solvent, a linear carbonate-based organic solvent, or a mixture thereof. At this time, the cyclic carbonate-based organic solvent and the linear carbonate-based organic solvent may be mixed in a volume ratio of 5:95 to 40:60, specifically, a volume ratio of 7:93 to 30:70. When the mixing ratio of the cyclic carbonate-based organic solvent and the linear carbonate-based organic solvent satisfies the above-mentioned range, high dielectric constant and low viscosity characteristics may be simultaneously satisfied, and excellent ionic conductivity characteristics may be implemented.

In addition, the organic solvent may further include, in addition to at least one carbonate-based organic solvent selected from the group consisting of the cyclic carbonate-based organic solvent and the linear carbonate-based organic solvent, at least one ester-based organic solvent selected from the group consisting of a linear ester-based organic solvent and a cyclic ester-based organic solvent, in order to produce an electrolyte having high ionic conductivity.

Specifically, the linear ester-based organic solvent may include at least one selected from the group consisting of methyl acetate, ethyl acetate, propyl acetate, methyl propionate, ethyl propionate, propyl propionate, and butyl propionate.

In addition, specifically, the cyclic ester-based organic solvent may include at least one selected from the group consisting of γ-butyrolactone, γ-valerolactone, γ-caprolactone, σ-valerolactone, and ε-caprolactone.

Meanwhile, the organic solvent may be used without limitation by adding organic solvents commonly used in non-aqueous electrolytes as needed. For example, the organic solvent may additionally include at least one organic solvent selected from an ether-based organic solvent, a glyme-based solvent, and a nitrile-based organic solvent.

The ether-based solvent may include any one selected from the group consisting of dimethyl ether, diethyl ether, dipropyl ether, methyl ethyl ether, methyl propyl ether, ethyl propyl ether, 1,3-dioxolane (DOL), and 2,2-bis(trifluoromethyl)-1,3-dioxolane (TFDOL), or a mixture of two or more thereof, but is not limited thereto.

The glyme-based solvent has a high dielectric constant and low surface tension compared to the linear carbonate-based solvent, and has low reactivity with metals, and may include at least one selected from the group consisting of dimethoxyethane (glyme, DME), diethoxyethane, diglyme, tri-glyme, and tetra-glyme (TEGDME), but is not limited thereto.

The nitrile-based solvent may include at least one selected from the group consisting of acetonitrile, propionitrile, butyronitrile, valeronitrile, caprylonitrile, heptanenitrile, cyclopentane carbonitrile, cyclohexane carbonitrile, 2-fluorobenzonitrile, 4-fluorobenzonitrile, difluorobenzonitrile, trifluorobenzonitrile, phenylacetonitrile, 2-fluorophenylacetonitrile, and 4-fluorophenylacetonitrile, but is not limited thereto.

(3) Additive

The additive includes a compound represented by Formula 1:

(Formula 1)

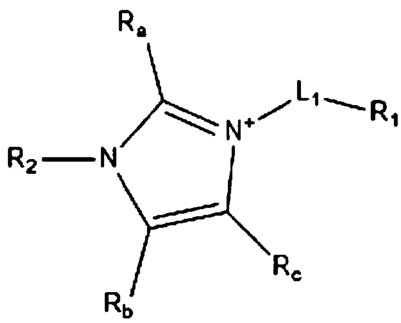

In Formula 1, $R_1$ is a substituent represented by Formula 2 below, $L_1$ is a direct bond or a substituted or unsubstituted alkylene group having 1 to 6 carbon atoms, $R_2$ is hydrogen or a substituent represented by Formula 3 below, and $R_a$, $R_b$, and $R_c$ are each independently hydrogen, an alkyl group having 1 to 3 carbon atoms, or -CN.

(Formula 2)

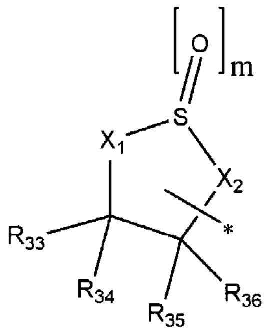

In Formula 2, m is 1 or 2, $X_1$ and $X_2$ are each independently -O- or -C($R_{31}$)($R_{32}$)-, provided that at least one of $X_1$ and $X_2$ is -O-, $R_{31}$ to $R_{36}$ are each independently hydrogen, an alkyl group having 1 to 6 carbon atoms, -C(=O)-$R_4$, or -$R_5$-O-C(=O)-$R_6$, $R_4$ and $R_6$ are each independently a substituted or unsubstituted alkyl group having 1 to 6 carbon atoms, an alkenyl group having 2 to 20 carbon atoms, an alkynyl group having 2 to 20 carbon atoms, or a substituted or unsubstituted aryl group having 6 to 20 carbon atoms, $R_5$ is a substituted or unsubstituted alkylene group having 1 to 6 carbon atoms, each of substituents of $L_1$, $R_4$, $R_5$, and $R_6$ are each independently at least one selected from the group consisting of deuterium, -F, -Cl, -Br, -I, -CN, -$NO_2$, and -$SO_3$, and * is a bonding site located at one of $R_{31}$, $R_{32}$, $R_{33}$, $R_{34}$, $R_{35}$, and $R_{36}$.

(Formula 3)

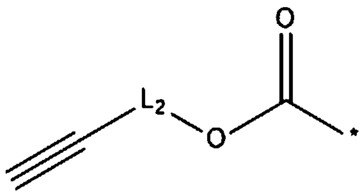

In Formula 3, * is a bonding site, and $L_2$ is an alkylene group having 1 to 3 carbon atoms.

The compound represented by Formula 1 includes, as an additive, a compound of a specific chemical formula having an imidazolium cation structure substituted with a cyclic sulfur oxide. The compound is a hetero compound having a cyclic structure in which carbon and nitrogen exist in the structure, and since a nitrogen element rich in electrons exists in the structure, electrons may be evenly arranged in the cyclic structure, so that the cyclic structure of the cyclic sulfur oxide may be maintained without being immediately opened during charge and discharge. The compound represented by Formula 1 having such characteristics may continuously contribute to the formation of films on the positive and negative electrodes during long-term charge and discharge. Particularly, in the case of a positive electrode having a high energy density or a positive electrode requiring high-voltage operation, a decrease in the thermal stability of the positive electrode or an electrolyte side reaction at the positive electrode is problematic, which may be caused by a decrease in the durability of the film or a breakage of the film during long-term operation of the positive electrode. The compound represented by Formula 1 may continuously contribute to the formation of the positive electrode film during charge and discharge of the lithium secondary battery, which may be expected to improve long-term life performance, high-temperature durability, and thermal stability. Accordingly, a lithium secondary battery including the non-aqueous electrolyte described above may have excellent life performance and high-temperature storage characteristics.

In addition, since the compound represented by Formula 1 has an imidazolium cation in its parent structure, the generation of Lewis acids such as HF and $PF_5$ may be suppressed, while the nitrogen element acts as a Lewis base to remove the Lewis acid generated in the electrolyte, so that the deterioration behavior of the film on the surface of the positive or negative electrode caused by the Lewis acid may be suppressed, thereby preventing additional electrolyte decomposition. As a result, self-discharge of a lithium secondary battery may be alleviated, thereby improving high-temperature storage characteristics.

In Formula 1, $R_1$ is a substituent represented by Formula 2:

(Formula 2)

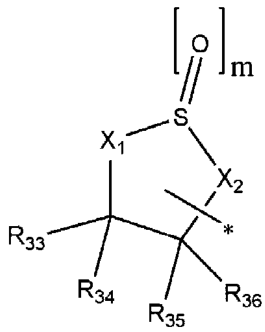

In the formula 2, m is an integer of 1 to 2. Specifically, m may be 2.

$X_1$ and $X_2$ are each independently -O- or -C($R_{31}$)($R_{32}$)-, provided that at least one of $X_1$ and $X_2$ is -O-. Specifically, $X_1$ may be -O- and/or $X_2$ may be -C($R_{31}$)($R_{32}$)-.

$R_{31}$ to $R_{36}$ may be each independently hydrogen, an alkyl group having 1 to 6 carbon atoms, -C(=O)-$R_4$, or -$R_5$-O-C(=O)-$R_6$. $R_4$ and $R_6$ may be each independently a substituted or unsubstituted alkyl group having 1 to 6 carbon atoms, specifically, a substituted or unsubstituted alkyl group having 1 to 3 carbon atoms, an alkenyl group having 2 to 20 carbon atoms, specifically, an alkenyl group having 2 to 5 carbon atoms, an alkynyl group having 2 to 20 carbon atoms, specifically, an alkynyl group having 2 to 5 carbon atoms, or a substituted or unsubstituted aryl group having 6 to 20 carbon atoms, specifically, a substituted or unsubstituted aryl group having 6 to 10 carbon atoms. $R_5$ is a substituted or unsubstituted alkylene group having 1 to 6 carbon atoms, specifically, a substituted or unsubstituted alkylene group having 1 to 3 carbon atoms. When substituents are present at $L_1$, $R_4$, $R_5$, and $R_6$, the substituents of $L_1$, $R_4$, $R_5$, and $R_6$ may be each independently at least one selected from the group consisting of deuterium, -F, - Cl, -Br, -I, -CN, -$NO_2$, and -$SO_3$. Specifically, $R_{31}$ to $R_{36}$ may be hydrogen.

In Formula 2, * is a bonding site and may be located at one of $R_{31}$, $R_{32}$, $R_{33}$, $R_{34}$, $R_{35}$, and $R_{36}$, When * is located at $R_{31}$, $R_{32}$, $R_{33}$, $R_{34}$, $R_{35}$, or $R_{36}$, it may mean that hydrogen or another substituent does not exist at $R_{31}$, $R_{32}$, $R_{33}$, $R_{34}$, $R_{35}$, or $R_{36}$, and the carbon adjacent to $R_{31}$, $R_{32}$, $R_{33}$, $R_{34}$, $R_{35}$, or $R_{36}$ is directly bonded to $L_1$.

The substituent represented by Formula 2 may be any one selected from substituents CS-1 to CS-15. When the substituents of CS-1 to CS-15 are applied as $R_1$ in Formula 1, the overall compound has excellent structural stability and may smoothly perform its function as an additive. Specifically, the substituent represented by Formula 2 may be any one selected from the group consisting of CS-1, CS-2, CS-5, CS-8, CS-10, and CS-11 in terms of structural stability and ease of synthesis. More specifically, the substituent represented by Formula 2 may be CS-8.

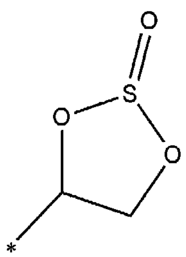
CS-1

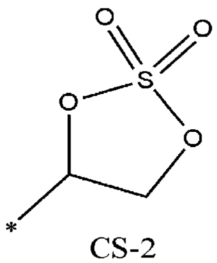
CS-2

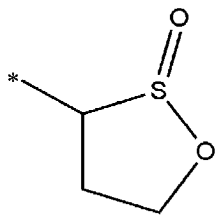
CS-3

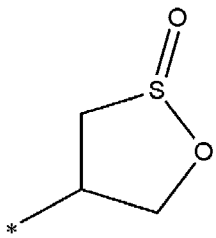
CS-4

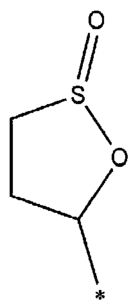
CS-5

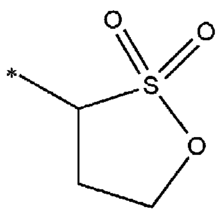
CS-6

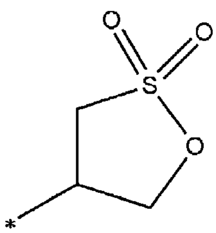
CS-7

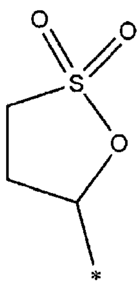
CS-8

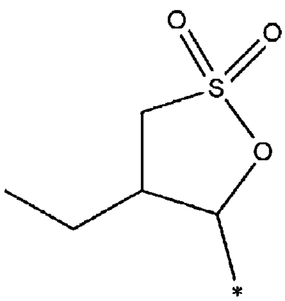
CS-9

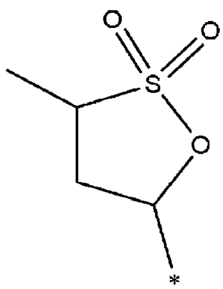
CS-10

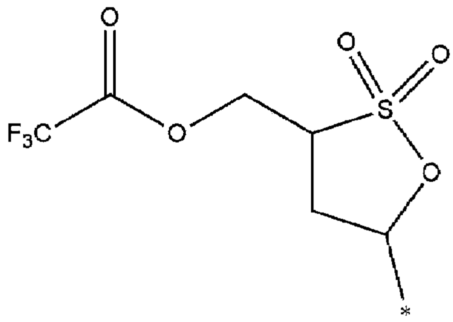

CS-11

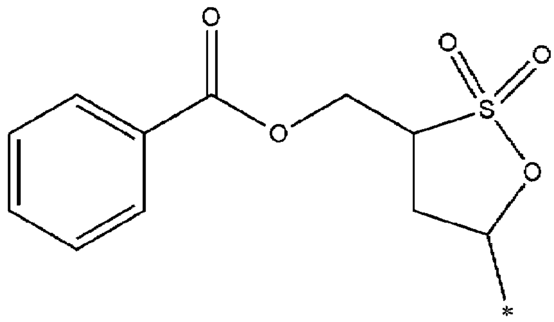

CS-12

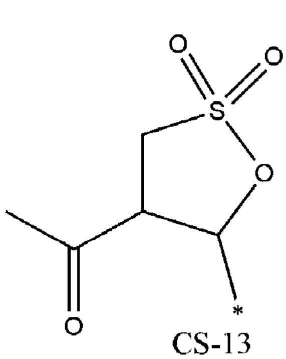

CS-13

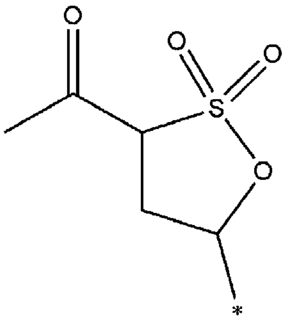

CS-14

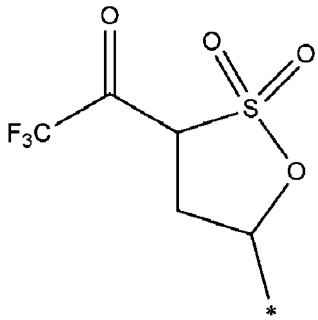

CS-15

In Formula 1, $L_1$ may be a direct bond, or a substituted or unsubstituted alkylene group having 1 to 6 carbon atoms, specifically, may be a direct bond, a methylene group, or an ethylene group, more specifically, may be a methylene group in terms of facilitating the synthesis of the compound and suppressing the decomposition of the compound after synthesis.

In Formula 1, $R_2$ may be hydrogen or a substituent represented by Formula 3:

(Formula 3)

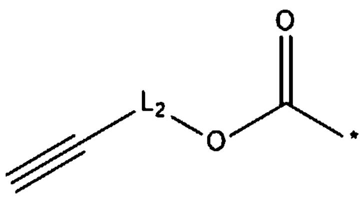

The substituent represented by Formula 3 contains a propargyl functional group that is easily reduced at the terminal, so that an electrode film having high passivation ability may be formed, thereby preventing additional reduction decomposition reactions caused by instability of the film, and improving the high-temperature durability of the electrode. In addition, the propargyl group contained in the substituent represented by Formula 3 may be adsorbed on the surface of metallic impurities included in the positive electrode, which may suppress the elution of the impurities, thereby suppressing the deposition of metal ions on the negative electrode surface, and thus, preventing internal short circuits.

$L_2$ may be an alkylene group having 1 to 3 carbon atoms, specifically, a methylene group, more specifically, an ethylene group, or a methylene group.

In Formula 1, $R_a$, $R_b$, and $R_c$ may each independently be hydrogen, an alkyl group having 1 to 3 carbon atoms, or -CN, for example, may each independently be hydrogen or an alkyl group having 1 to 2 carbon atoms, or may each be hydrogen.

Specifically, the compound represented by Formula 1 may include at least one of compounds represented by Formulas 1-1 and 1-2 below. Morespecifically, the compound represented by Formula 1 may include a compound represented by Formula 1-1 and a compound represented by Formula 1-2:

(Formula 1-1)

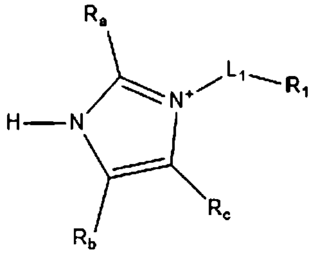

(Formula 1-2)

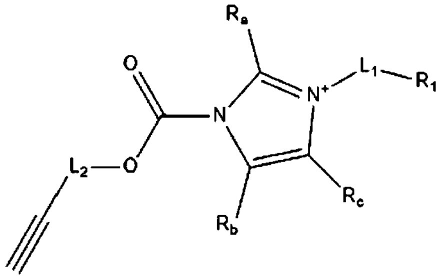

In Formulas 1-1 and 1-2, $R_a$, $R_b$, $R_c$, $R_1$, $L_1$, and $L_2$ are as defined in Formula 1.

Specifically, The compound represented by Formula 1 may include at least one of compounds represented by Formulas 1-A and 1-B below. Morespecifically, the compound represented by Formula 1 may include a compound represented by Formula 1-A and a compound represented by Formula 1-B:

(Formula 1-A)

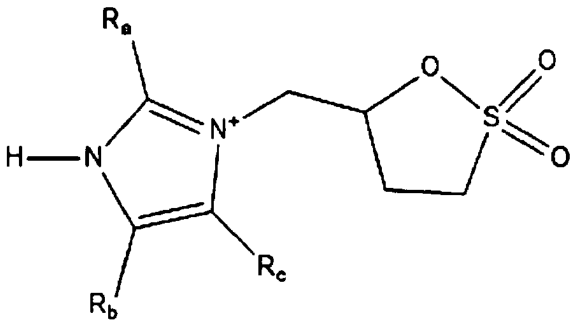

(Formula 1-B)

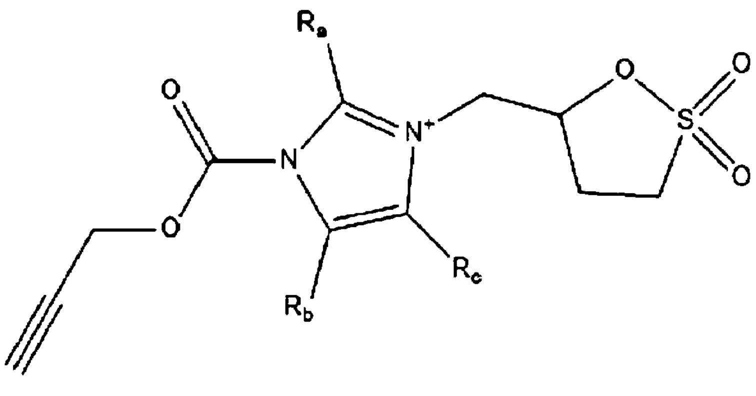

In Formulas 1-A and 1-B, $R_a$, $R_b$, and $R_c$ are as defined in Formula 1.

Specifically, the compound represented by Formula 1 may include at least one of compounds represented by Formulas 1-a and 1-b below. More specifically, the compound represented by Formula 1 may include a compound represented by Formula 1-a and a compound represented by Formula 1-b:

(Formula 1-a)

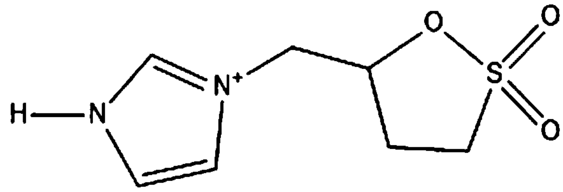

(Formula 1-b)

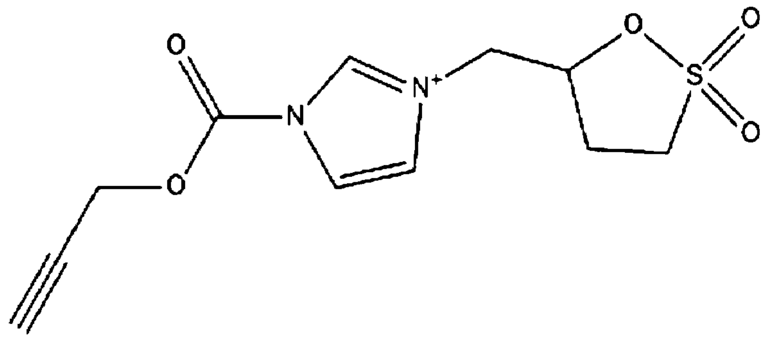

The compound represented by Formula 1 may be included in the non-aqueous electrolyte in an amount of 0.01 wt% to 10 wt%, specifically, in an amount of 0.1 wt% to 5 wt%, more specifically, in an amount of 0.5 wt% to 3 wt%, more specifically, in an amount of 0.2 wt% to 1 wt%, more specifically, may be included in an amount of 0.5 wt% to 1 wt%. When the compound is included in the non-aqueous electrolyte within the above range, the effect of improving high-temperature durability of the secondary battery described above may be exhibited, while preventing an increase in resistance due to excessive use of additives.

The additive may further include, together with the compound represented by Formula 1, at least one compound selected from the group consisting of a compound represented by Formula 4 below and a compound represented by Formula 5 below. Specifically, the additive may further include, together with the compound represented by Formula 1, a compound represented by Formula 4 below and a compound represented by Formula 5 below.

The additive may further include a compound represented by Formula 4:

(Formula 4)

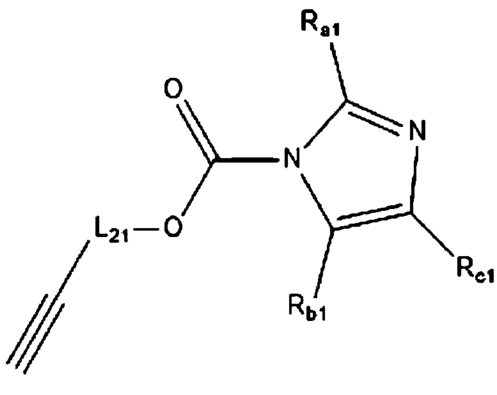

In Formula 4, $R_{a1}$, $R_{b1}$, and $R_{c1}$ are each independently hydrogen, an alkyl group having 1 to 3 carbon atoms, or -CN, and $L_{21}$ is an alkylene group having 1 to 3 carbon atoms.

In addition, the compound represented by Formula 4 is able to inhibit the generation of the Lewis acids such as HF and $PF_5$ by the imidazole group contained therein, while the nitrogen element is able to act as a Lewis base to remove the Lewis acids generated in the electrolyte, thereby inhibiting the degradation behavior of the film on the surface of the positive electrode or negative electrode caused by the Lewis acids, and thereby preventing further electrolyte degradation. As a result, self-discharge of a lithium secondary battery may be alleviated, thereby improving high-temperature storage characteristics.

In addition, the compound represented by Formula 4 contains a propargyl functional group that is easily reduced at the terminal, so that an electrode film having high passivation ability may be formed, thereby preventing or suppressing additional reduction decomposition reactions caused by instability of the film, and improving the high-temperature durability of the electrode. In addition, the propargyl group contained in the compound represented by Formula 4 may be adsorbed on the surface of metallic impurities included in the positive electrode, which may suppress the elution of the impurities, thereby preventing or suppressing the deposition of metal ions on the negative electrode surface, and thus, preventing internal short circuits.

In Formula 4, $R_{a1}$, $R_{b1}$, and $R_{c1}$ may each independently be hydrogen, an alkyl group having 1 to 3 carbon atoms, or -CN, specifically, may each independently be hydrogen, or an alkyl group having 1 to 2 carbon atoms, more specifically, may each be hydrogen.

Specifically, $L_{21}$ may be an alkylene group having 1 to 3 carbon atoms, for example, may be a methylene group or an ethylene group, or may be a methylene group.

The compound represented by the formula 4 may include a compound represented by Formula 4-1:

(Formula 4-1)

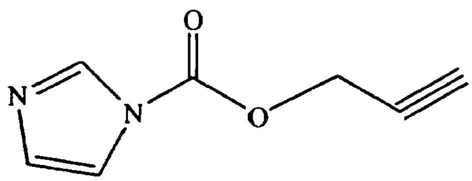

When the compound represented by Formula 4 is included in the non-aqueous electrolyte, the compound represented by Formula 4 may be included in the non-aqueous electrolyte in an amount of 0.01 wt% to 10 wt%, specifically, may be included in the non-aqueous electrolyte in an amount of 0.1 wt% to 5 wt%.

The additive may further include a compound represented by Formula 5:

(Formula 5)

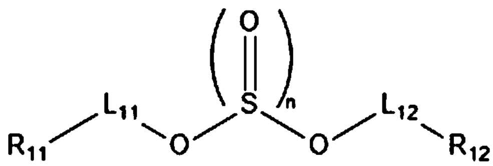

In Formula 5, n is 1 or 2, $L_{11}$ and $L_{12}$ are each independently a direct bond or a substituted or unsubstituted alkylene group having 1 to 6 carbon atoms, and $R_{11}$ and $R_{12}$ are each independently a substituent represented by Formula 6:

(Formula 6)

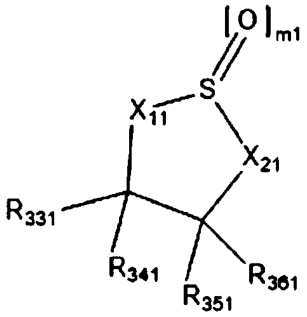

In Formula 6, m1 is 1 or 2, $X_{11}$ and $X_{21}$ are each independently -O- or - $C(R_{311})(R_{321})$-, provided that at least one of $X_{11}$ and $X_{21}$ is -O-; $R_{311}$, $R_{321}$, $R_{331}$, $R_{341}$, $R_{351}$, and $R_{361}$ are each independently hydrogen, an alkyl group having 1 to 6 carbon atoms, - $C(=O)-R_{41}$, or $-R_{51}-O-C(=O)-R_{61}$; $R_{41}$ and $R_{61}$ are each independently a substituted or unsubstituted alkyl group having 1 to 6 carbon atoms, an alkenyl group having 2 to 20 carbon atoms, an alkynyl group having 2 to 20 carbon atoms, or substituted or unsubstituted aryl group having 6 to 20 carbon atoms; $R_{51}$ is a substituted or unsubstituted alkylene group having 1 to 6 carbon atoms; each of substituents of $L_{11}$, $L_{21}$, $R_{41}$, $R_{51}$, and $R_{61}$ are each independently at least one selected from the group consisting of deuterium, -F, -Cl, -Br, -I, -CN, $-NO_2$, and $-SO_3$; * is a bonding site located at one of $R_{311}$, $R_{321}$, $R_{331}$, $R_{341}$, $R_{351}$, and $R_{361}$; when $L_{11}$ and $L_{21}$ are both direct bonds, $R_{11}$ and $R_{21}$ are not CS1-7 below at the same time; and when $L_{11}$ and $L_{21}$ are both methylene groups and n is 2, $R_{11}$ and $R_{21}$ are not CS1-2 below at the same time:

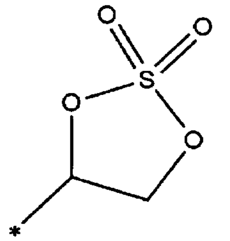

CS1-2

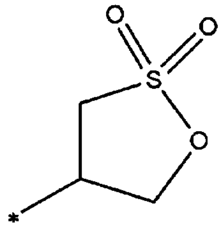

CS1-7

The compound represented by Formula 5 includes a sulfur oxide structure at the center, while at least one of both terminals has a cyclic sulfur oxide structure. By employing this chemical structure, when applied as a non-aqueous electrolyte additive, the compound may induce stable formation of anions, and further, may enable the formation of a stable solid electrolyte interphase (SEI) layer.

In Formula 5, n is 1 or 2, and may be, specifically, 2.

$L_{11}$ and $L_{12}$ may each independently be a direct bond or a substituted or unsubstituted alkylene group having 1 to 6 carbon atoms, specifically, may each independently be a methylene group or an ethylene group, more specifically, may each be a methylene group.

$R_{11}$ and $R_{12}$ are each independently a substituent represented by Formula 6.

In Formula 6, m is 1 or 2, and may be, specifically, 2.

$X_{11}$ and $X_{21}$ are each independently -O- or $-C(R_{311})(R_{321})$-, provided that at least one of $X_{11}$ and $X_{21}$ is -O-. For example, $X_{11}$ may be -O- and/or $X_{21}$ may be - $C(R_{311})(R_{321})$-.

$R_{311}$, $R_{321}$, $R_{331}$, $R_{341}$, $R_{351}$, and $R_{361}$ may be each independently hydrogen, an alkyl group having 1 to 6 carbon atoms, $-C(=O)-R_{41}$, or $-R_{51}-O-C(=O)-R_{61}$. $R_{41}$ and $R_{61}$ may be each independently a substituted or unsubstituted alkyl group having 1 to 6 carbon atoms, specifically, a substituted or unsubstituted alkyl group having 1 to 3 carbon atoms, an alkenyl group having 2 to 20 carbon atoms, specifically, an alkenyl group having 2 to 5 carbon atoms, an alkynyl group having 2 to 20 carbon atoms, specifically, an alkynyl group having 2 to 5 carbon atoms, or a substituted or unsubstituted aryl group having 6 to 20 carbon atoms, specifically, a substituted or unsubstituted aryl group having 6 to 10 carbon atoms. $R_{51}$ is a substituted or unsubstituted alkylene group having 1 to 6 carbon atoms, specifically, a substituted or unsubstituted alkylene group having 1 to 3 carbon atoms. When substituents are present at $L_{11}$, $R_{41}$, $R_{51}$, and $R_{61}$, the substituents of $L_{11}$, $R_{41}$, $R_{51}$, and $R_{61}$ may be each independently at least one selected from the group consisting of deuterium, -F, -Cl, -Br, -I, -CN, $-NO_2$, and $-SO_3$. Specifically, $R_{311}$, $R_{321}$, $R_{331}$, $R_{341}$, $R_{351}$, and $R_{361}$ may be hydrogen.

In Formula 6, * is a bonding site and may be located at one of $R_{311}$, $R_{321}$, $R_{331}$, $R_{341}$, $R_{351}$, and $R_{361}$. When * is located at $R_{311}$, $R_{321}$, $R_{331}$, $R_{341}$, $R_{351}$, or $R_{361}$, it may mean that hydrogen or another substituent does not exist at $R_{311}$,

$R_{321}$, $R_{331}$, $R_{341}$, $R_{351}$, or $R_{361}$, and the carbon adjacent to $R_{311}$, $R_{321}$, $R_{331}$, $R_{341}$, $R_{351}$, or $R_{361}$ is directly bonded to $L_{11}$ or $L_{21}$.

The substituent represented by Formula 6 may be any one selected from substituents CS1-1 to CS1-15: When the substituents of CS1-1 to CS1-15 are applied as $R_{11}$ or $R_{12}$ in Formula 6, the overall compound has excellent structural stability and may smoothly perform its function as an additive. Specifically, the substituent represented by Formula 6 may be any one selected from the group consisting of CS1-1, CS1-2, CS1-5, CS1-8, CS1-10, and CS1-11 in terms of structural stability and ease of synthesis. More specifically, the substituent represented by Formula 6 may be CS1-8.

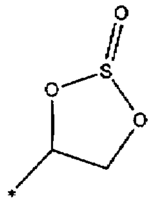
CS1-1

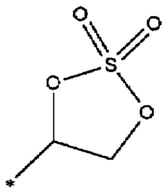
CS1-2

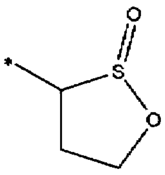
CS1-3

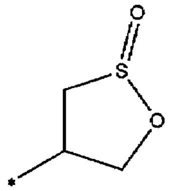
CS1-4

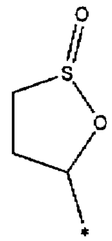
CS1-5

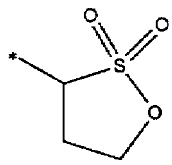
CS1-6

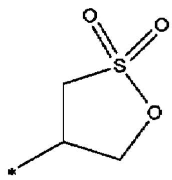
CS1-7

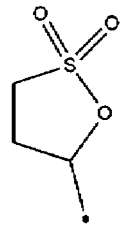
CS1-8

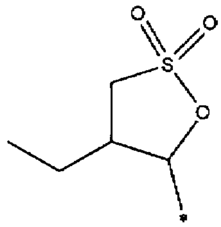
CS1-9

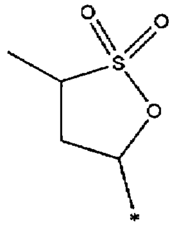
CS1-10

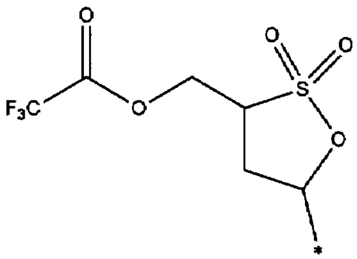
CS1-11

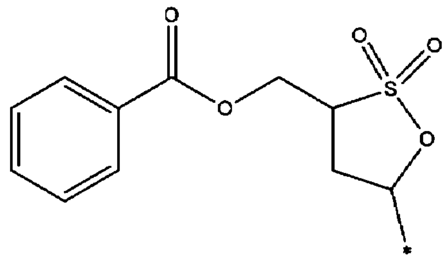
CS1-12

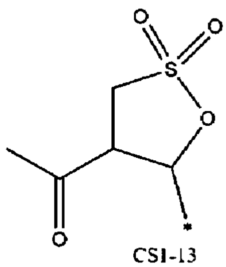
CS1-13

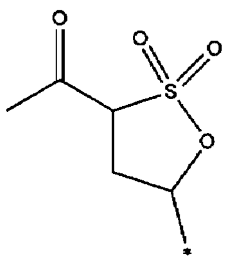
CS1-14

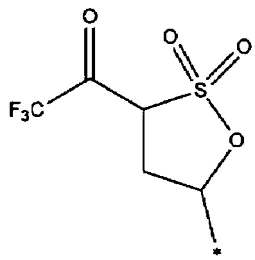
CS1-15

More specifically, the compound represented by Formula 5 may include at least one compound selected from the group consisting of A to Q below. More specifically, the compound represented by Formula 5 may include at least one compound selected from the group consisting of compound A, compound F, and compound J below. More specifically, the compound represented by Formula 5 may include the compound represented by Formula A.

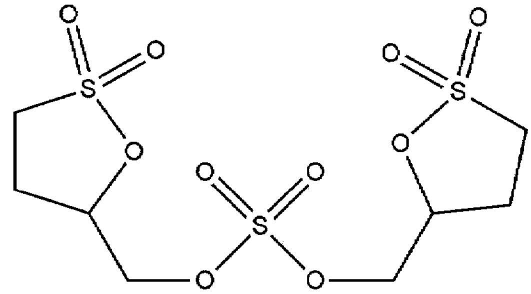

A

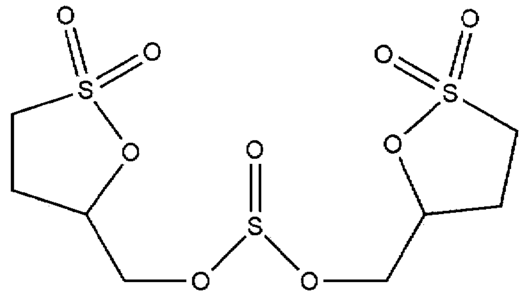

B

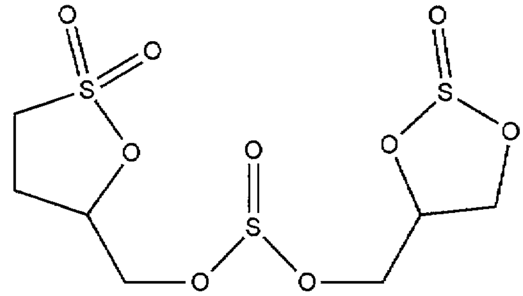

C

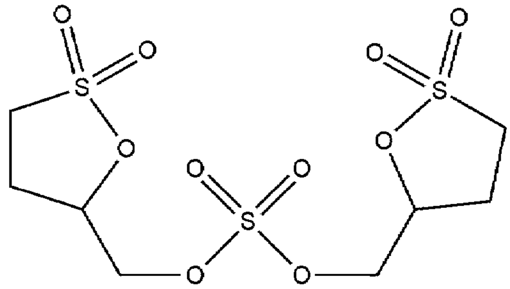

D

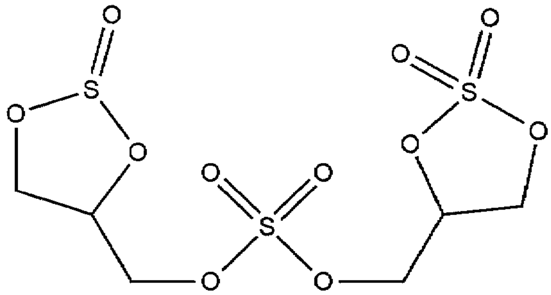

E

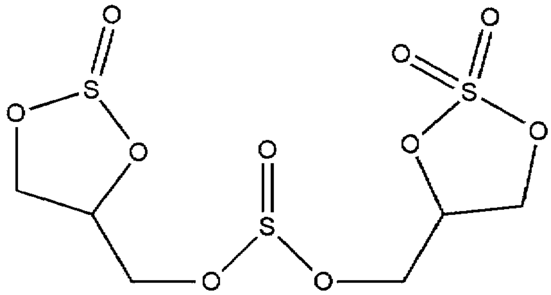

F

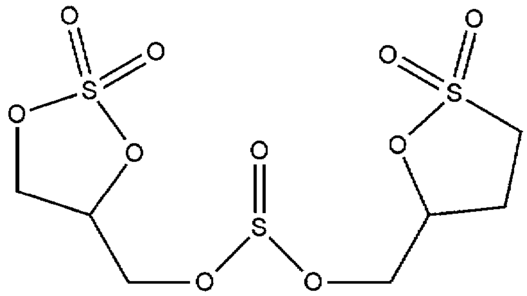

G

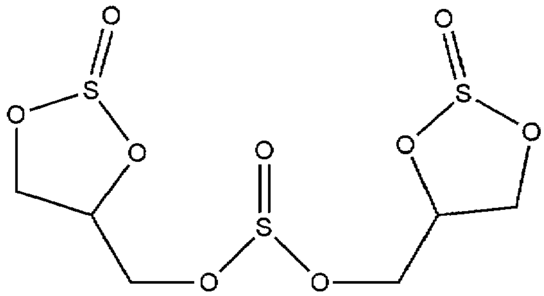

H

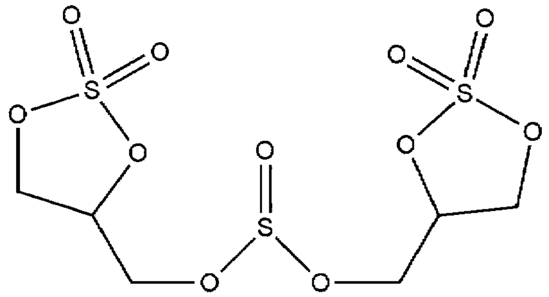

I

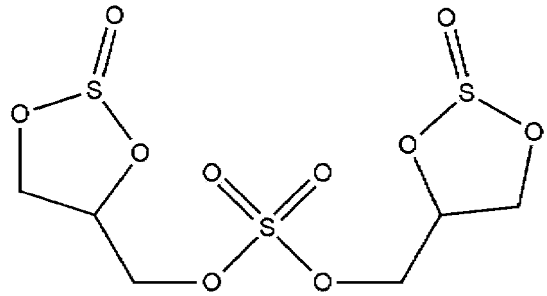

J

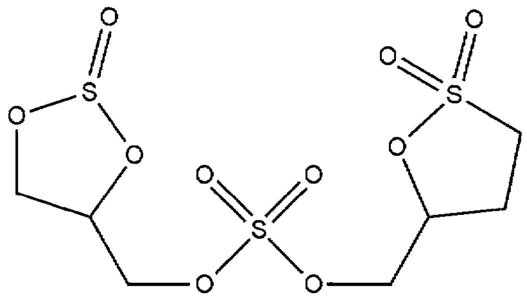

K

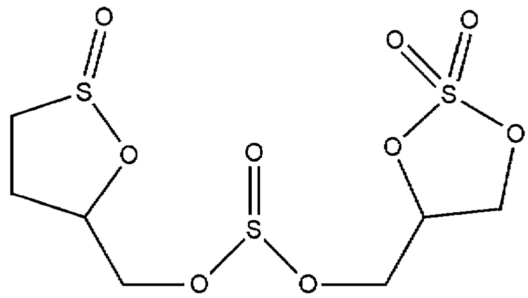

L

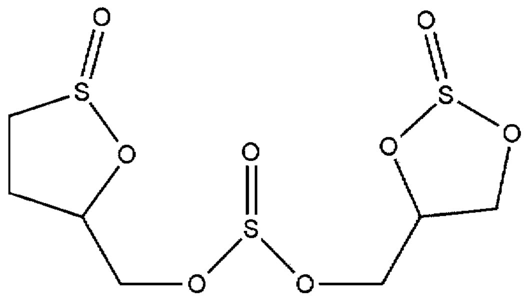

M

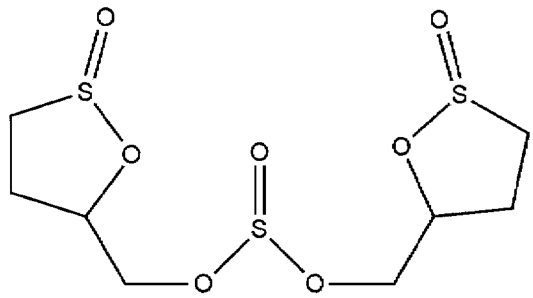

N

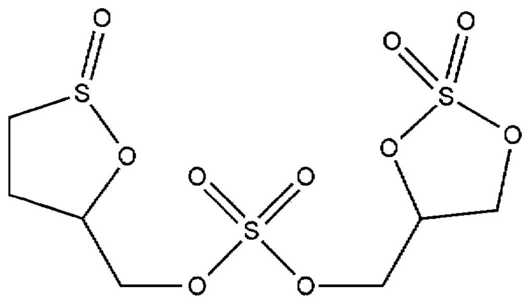

O

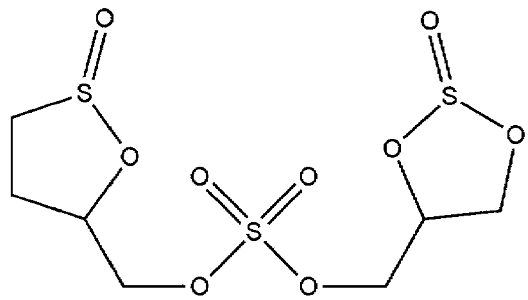

P

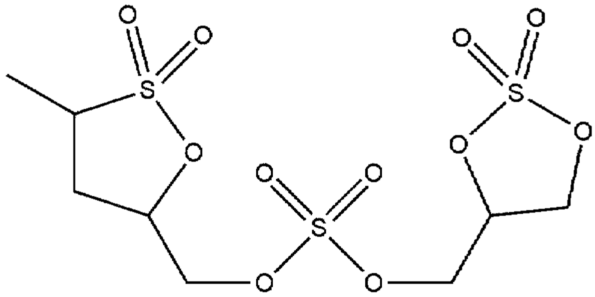

Q

When the compound represented by Formula 5 is included in the non-aqueous electrolyte, the compound represented by Formula 5 may be included in the non-aqueous electrolyte in an amount of 0.01 wt% to 10 wt%, specifically, 0.1 wt% to 5 wt%.

The method for preparing the compound represented by Formula 1 is not particularly limited, and may be prepared, for example, by reacting an imidazole compound with a cyclic sulfur oxide compound capable of reacting with the imidazole compound to bond a cyclic sulfur oxide functional group to a nitrogen position of the imidazole compound, thereby forming an imidazolium cation compound. For example, the compound represented by Formula 1 may be prepared by reacting the compound represented by Formula 4 and the compound represented by Formula 5 to form a compound in the form of an imidazolium cation substituted with a cyclic sulfur oxide. The compound represented by Formula 5 has $-SO_4-$, $-SO_3-$, or the like, which may serve as a leaving group, bonded to the cyclic sulfur oxide ($R_{11}$ or $R_{12}$), thereby facilitating reaction with the nitrogen of the imidazole group in the compound represented by Formula 4. The compound represented by Formula 4 and the compound represented by Formula 5 may all form a compound represented by Formula 1, or some of these compounds may form a compound represented by Formula 1, depending on the adjustment of the equivalent amount during the reaction. Meanwhile, when the compound represented by Formula 4 reacts with 1,3-propane sultone (PS), the unshared electron pair of imidazole opens the ring of the 1,3-propane sultone, which makes it difficult to implement the compound of Formula 1.

More specifically, the compound represented by Formula 1 may be prepared or formed by adding the compound represented by Formula 4 and the compound represented by Formula 5 to an organic solvent or a non-aqueous electrolyte, and then allowing a spontaneous reaction of these compounds. Alternatively, the compound represented by Formula 1 may be prepared or formed by adding the compound represented by Formula 4 and the compound represented by Formula 5 to an organic solvent or a non-aqueous electrolyte, and then sufficiently aging or leaving at room temperature (15°C to 25°C) to allow the compounds to react.

The additive may further include an additional additive together with the compound represented by Formula 1. The additional additive may be included in the non-aqueous electrolyte to prevent the non-aqueous electrolyte from decomposing and causing negative electrode collapse in high power environments, or for low temperature high rate discharge characteristics, high temperature stability, overcharge protection, and battery expansion inhibition at high temperatures.

Specifically, the additional additive may include at least one selected from the group consisting of lithium difluorophosphate (LiDFP), vinylene carbonate, vinyl ethylene carbonate, fluoroethylene carbonate, propane sultone, propene sultone, succinonitrile, adiponitrile, ethylene sulfate, lithium bis-(oxalato)borate (LiBOB), 3-trimethoxysilanyl-propyl-N-aniline (TMSPa), and tris(trimethylsilyl) phosphite (TMSPi), and specifically, may include lithium difluorophosphate (LiDFP).

The additional additive may be included in the negative electrode active material in an amount of 0.1 wt% to 15 wt%, specifically, 0.3 wt% to 10 wt%.

## Lithium Secondary Battery

The present invention also provides a lithium secondary battery. Specifically, the lithium secondary battery may include the non-aqueous electrolyte described above.

More specifically, the lithium secondary battery according to the present invention includes a positive electrode; a negative electrode opposite to the positive electrode; a separator interposed between the positive electrode and the negative electrode; and the non-aqueous electrolyte described above.

The lithium secondary battery may be manufactured by accommodating an electrode assembly including the positive electrode; a negative electrode opposite the positive electrode; and a separator interposed between the positive electrode and the negative electrode, in a battery case, and then injecting the above-described non-aqueous electrolyte

thereinto.

(1) Positive Electrode

The positive electrode may include a positive electrode active material.

The positive electrode active material is a compound capable of reversible intercalation and deintercalation of lithium, and specifically, may include a lithium-transition metal composite oxide including lithium and at least one transition metal selected from nickel, cobalt, manganese, and aluminum, more specifically, a lithium-transition metal composite oxide including lithium and a transition metal selected from nickel, cobalt, and manganese.

For example, the lithium transition metal composite oxides include lithium-manganese oxides (*e.g.,* $LiMnO_2$ and $LiMn_2O_4$), lithium-cobalt oxides (*e.g.,* $LiCoO_2$), lithium-nickel oxides (*e.g.,* $LiNiO_2$), lithium-nickel-manganese oxides (*e.g.,* $LiNi_{1-Y}Mn_YO_2$ (wherein, 0<Y<1) and $LiMn_{2-Z}Ni_zO_4$ (wherein, 0<Z<2)), lithium-nickel-cobalt oxides (*e.g.,* $LiNi_{1-Y1}Co_{Y1}O_2$ (wherein, 0<Y1<1)), lithium-manganese-cobalt oxides (*e.g.,* $LiCo_{1-Y2}Mn_{Y2}O_2$ (wherein, 0<Y2<1), $LiMn_{2-z1}Co_{z1}O_4$ (wherein, 0<Z1<2)), lithium-nickel-manganese-cobalt oxides (*e.g.*, $Li(Ni_pCo_qMn_{r1})O_2$ (wherein, 0 < p < 1, 0 < q < 1, 0< r1 < 1, p+q+r1=1) or $Li(Ni_{p1}Co_{q1}Mn_2)O_4$ (wherein, 0 < p1 < 2, 0 < q1 < 2, 0 < r2 < 2, p1+q1+r2=2)), or lithium-nickel-cobalt-transition metal (M) oxide (*e.g.,* $Li(Ni_{p2}Co_{q2}Mn_{r3}Ms_2)O_2$ (wherein, M is selected from Al, Fe, V, Cr, Ti, Ta, Mg, and Mo, and p2, q2, r3 and s2 are atomic fractions of independent elements and satisfy 0 < p2 < 1, 0 < q2 < 1, 0 < r3 < 1, 0 < s2 < 1, p2+q2+r3+s2=1)), and may include one or more compounds thereof. Among these, in terms of being able to improve the capacity characteristics and stability of the battery, the lithium transition metal composite oxide may be $LiCoO_2$, $LiMnO_2$, $LiNiO_2$, lithium nickel-manganese-cobalt oxide (*e.g.,* $Li(Ni_{0.6}Mn_{0.2}Co_{0.2})O_2$, $Li(Ni_{0.5}Mn_{0.3}Co_{0.2})O_2$, $Li(Ni_{0.7}Mn_{0.15}Co_{0.15})O_2$, or $Li(Ni_{0.8}Mn_{0.1}Co_{0.1})O_2$), or lithium nickel-cobalt-aluminum oxide (*e.g.,* $Li(Ni_{0.8}Co_{0.15}Al_{0.05})O_2$), and considering the prominence of the improvement effect according to the control of the type and content ratio of the constituent elements forming the lithium transition metal composite oxide, the lithium transition metal composite oxide may be $Li(Ni_{0.6}Mn_{0.2}Co_{0.2})O_2$, $Li(Ni_{0.5}Mn_{0.3}Co_{0.2})O_2$, $Li(Ni_{0.7}Mn_{0.15}Co_{0.15})O_2$, or $Li(Ni_{0.8}Mn_{0.1}Co_{0.1})O_2$, which may be used either alone or in mixture of two or more thereof.

More specifically, the positive electrode active material may include a lithium transition metal oxide represented by the Formula P-1:

(Formula P-1) $Li_{1+x}[Ni_aCo_bMn_cM^1{}_d]O_{2+w}$

In Formula P-1, x, a, b, c, d, and w satisfy 0≤x≤0.5, a+b+c+d = 1, 0.5≤a≤0.7, 0≤b≤0.15, c=1-a-b-d, 0≤d≤0.1, 0≤b/a≤0.2, 1≤a/c≤3, 0≤w≤1, respectively, and $M^1$ is at least one selected from W, Cu, Fe, V, Cr, Ti, Zr, Zn, Al, In, Ta, Y, La, Sr, Ga, Sc, Gd, Sm, Ca, Ce, Nb, Mg, B, and Mo.

The compound represented by Formula P-1 needs to be driven at a high voltage (*e.g.,* 4.35 V or higher) to increase the energy density of the positive electrode because the nickel content is lower than that of the high-nickel lithium transition metal oxide. However, during such high-voltage driving, the positive electrode electrolyte side reaction is intensified, so that the life performance and storage performance are greatly reduced, and the high-temperature durability is lowered, which aggravates the problem of increased resistance. This is because the oxidation and decomposition of organic solvents (*e.g.*, ethylene carbonate) are promoted during high-voltage driving to produce carbon dioxide ($CO_2$), or because Lewis acids such as HF and $PF_5$ formed by the decomposition of lithium salts (*e.g.*, $LiPF_6$) decompose the positive electrode film and elute the transition metal of the positive electrode active material thereby causing structural collapse. This phenomenon may be suppressed by using the non-aqueous electrolyte described above. As described above, since the non-aqueous electrolyte described above may continuously provide a film that may improve the durability of the positive electrode, the problem of electrolyte side reactions caused during long-term charge and discharge is significantly prevented and thus, the lithium secondary battery according to the present invention may exhibit excellent effects in long-term life performance and high-temperature storage performance.

In Formula P-1, the symbol x may be in the range of 0≤x≤0.5, specifically,0≤x≤0.2.

In Formula P-1, the symbol a may be in the range of 0≤a≤0.7, specifically, 0.55≤a≤0.65.

In Formula P-1, the symbol b may be in the range of 0≤b≤0.15. The symbol b corresponds to the molar percentage of Co among the metals excluding lithium in the lithium transition metal oxide represented by Formula A. According to the present invention, by lowering the Co content, it is possible to have a cost advantage, and by relatively increasing the proportion of Mn, it is possible to improve the structural stability of the positive electrode active material. Specifically, in Formula P-1, the symbol b may be in the range of0≤b≤0.1.

In Formula P-1, according to one embodiment, b/a may satisfy the range of 0≤b/a≤0.2. When b/a exceeds 0.2, the ratio of Co in the transition metal may be very high, thereby increasing the irreversibility within the structure. Specifically, in Formula P-1, b/a may satisfy the range of 0.05≤b/a≤0.2.

In Formula P-1, c satisfies an equation of c=1-a-b-d, and a/c satisfies the range of 1≤a/c≤3. The symbol c corresponds to the molar percentage of Mn among the metals excluding lithium in the lithium transition metal oxide

represented by Formula P-1. According to the present invention, the molar ratio of Ni to Mn is adjusted to the range of $1 \leq a/c \leq 3$, thereby improving the structural stability of the positive electrode active material. Specifically, the molar ratio of Ni to Mn may be in the range of $1.5 \leq a/c \leq 2.5$.

In Formula P-1, $M^1$ may be understood as a doped element of lithium transition metal oxide, and may be, specifically, at least one selected from W, Cu, Fe, V, Cr, Ti, Zr, Zn, Al, In, Ta, Y, La, Sr, Ga, Sc, Gd, Sm, Ca, Ce, Nb, Mg, B, and Mo. Here, the symbol d may be in the range of $0 \leq d \leq 0.1$, specifically, $0 \leq d \leq 0.05$.

In another aspect, the positive electrode active material may include a lithium transition metal oxide represented by the Formula P-2:

(Formula P-2) $Li_{1+x1}(Ni_{a1}CO_{b1}Mn_{c1}M^2_{d1})O_2$

In Formula P-2, $M^2$ is at least one selected from the group consisting of W, Cu, Fe, V, Cr, Ti, Zr, Zn, Al, In, Ta, Y, La, Sr, Ga, Sc, Gd, Sm, Ca, Ce, Nb, Mg, B, and Mo, and 1+x1, a1, b1, c1, and d1 are atomic fractions of independent elements, respectively, and satisfy the ranges of $0 \leq x1 \leq 0.2$, $0.50 \leq a1 < 1$, $0 < b1 \leq 0.25$, $0 < c1 \leq 0.25$, $0 \leq d1 \leq 0.1$, and $a1+b1+c1+d1=1$.

specifically, each of the symbols a1, b1, c1, and d1 may satisfy the ranges of $0.70 \leq a1 \leq 0.95$, $0.025 \leq b1 \leq 0.20$, $0.025 \leq c1 \leq 0.20$, and $0 \leq d1 \leq 0.05$, respectively. In addition, the symbols a1, bl, c1, and d1 may satisfy the ranges of $0.80 \leq a1 \leq 0.95$, $0.025 \leq b1 \leq 0.15$, $0.025 \leq c1 \leq 0.15$, and $0 \leq d1 \leq 0.05$, respectively. In addition, the symbols a1, b1, c1, and d1 may satisfy the ranges of $0.85 \leq a1 \leq 0.90$, $0.05 \leq b1 \leq 0.10$, $0.05 \leq c1 \leq 0.10$, and $0 \leq d1 \leq 0.03$, respectively.

The positive electrode may include a positive electrode current collector, and a positive electrode active material layer disposed on at least one surface of the positive electrode current collector. At this time, the positive electrode active material may be included in the positive electrode active material layer.

The positive electrode current collector is not particularly limited as long as it has high conductivity without causing chemical changes in the battery. Specifically, the positive electrode current collector may include at least one selected from the group consisting of copper, stainless steel, aluminum, nickel, titanium, calcined carbon, and an aluminum-cadmium alloy, specifically, may include aluminum.

The thickness of the positive electrode current collector may have a thickness of 3 $\mu$m to 500 $\mu$m.

The positive electrode current collector may have fine unevenness formed on the surface to enhance the bonding strength of the positive electrode active material. For example, the positive electrode current collector may be used in various forms, such as a film, a sheet, a foil, a net, a porous body, a foam, and a non-woven fabric.

The positive electrode active material layer may be arranged on at least one side of the positive electrode current collector, specifically, on one or both sides of the positive electrode current collector.

The positive electrode active material may be included in the positive electrode active material layer in an amount of 80 wt% to 99 wt%, specifically, 92 wt% to 98.5 wt%, in consideration of sufficient capacity of the positive electrode active material.

Description of other positive electrode active materials is omitted as it has been described above.

The positive electrode active material layer may further include a binder and/or a conductive material together with the positive electrode active material.

The binder is a component that assists in the binding of the active material and the conductive material and the binding to the current collector, and may include, specifically, at least one selected from the group consisting of polyvinylidene fluoride, polyvinyl alcohol, carboxymethyl cellulose (CMC), starch, hydroxypropyl cellulose, regenerated cellulose, polyvinyl pyrrolidone, polytetrafluoroethylene, polyethylene, polypropylene, ethylene-propylene-diene terpolymer (EPDM), sulfonated EPDM, styrene-butadiene rubber, and fluororubber, more specifically, may include polyvinylidene fluoride.

The binder may be included in the positive electrode active material layer in an amount of 1 wt% to 20 wt%, specifically, 1.2 wt% to 10 wt%, in order to sufficiently secure binding force between components such as the positive electrode active material.

The conductive material may be used to assist and improve conductivity in a secondary battery, and is not particularly limited as long as it has conductivity without causing a chemical change. Specifically, the positive electrode conductive material may include at least one selected fromthe group consisting of graphite such as natural graphite or artificial graphite; carbon blacks such as carbon black, acetylene black, ketjenblack, channel black, furnace black, lamp black, and thermal black; conductive fibers such as carbon fibers or metal fibers; conductive tubes such as carbon nanotubes; fluorocarbons; metal powders such as aluminum or nickel powder; conductive whiskers such as zinc oxide or potassium titanate; conductive metal oxides such as titanium oxide; and polyphenylene derivatives, and specifically, may include carbon nanotubes in terms of improving conductivity.

The binder may be included in the positive electrode active material layer in an amount of 1 wt% to 20 wt%, specifically, 1.2 wt% to 10 wt%, in order to sufficiently secure electrical conductivity.

The thickness of the positive electrode active material layer may be 30 $\mu$m to 400 $\mu$m, specifically, 40 $\mu$m to 200 $\mu$m.

The positive electrode may be manufactured by coating a positive electrode slurry including a positive electrode active material and optionally a binder, a conductive material, and a solvent for forming the positive electrode slurry, on the positive electrode current collector, followed by drying and rolling.

The solvent for forming the positive electrode slurry may include an organic solvent such as NMP (N-methyl-2-pyrrolidone). The solid content of the positive electrode slurry may be 40 wt% to 90 wt%, specifically, may be about 50 wt% to 80 wt%.

(2) Negative Electrode

The negative electrode may be opposed to the positive electrode.

The negative electrode may include a negative electrode active material.

The negative active material is a material capable of reversibly inserting/removing lithium ions, and may include at least one selected from the group consisting of a carbon-based active material, a (semi)metal-based active material, and lithium metal, and specifically, may include at least one selected from a carbon-based active material and a (semi) metal-based active material.

The carbon-based active material may include at least one selected from the group consisting of graphite, hard carbon, soft carbon, carbon black, graphene, and fibrous carbon, and may include, specifically, graphite. The graphite may be, for example, at least one of artificial graphite and natural graphite.

The average particle diameter ($D_{50}$) of the carbon-based active material may be 10 μm to 30 μm, specifically, 15 μm to 25 μm, in terms of ensuring structural stability during charge and discharge and reducing side reactions with the electrolyte.

Specifically, the (semi)metal-based active material may include least one (semi)metal selected from the group consisting of Cu, Ni, Na, K, Rb, Cs, Fr, Be, Mg, Ca, Sr, Si, Sb, Pb, In, Zn, Ba, Ra, Ge, Al, V, Ti, and Sn; an alloy of lithium and at least one (semi)metal selected from the group consisting of Cu, Ni, Na, K, Rb, Cs, Fr, Be, Mg, Ca, Sr, Si, Sb, Pb, In, Zn, Ba, Ra, Ge, Al, V, Ti, and Sn; an oxide of at least one (semi)metal selected from the group consisting of Cu, Ni, Na, K, Rb, Cs, Fr, Be, Mg, Ca, Sr, Si, Sb, Pb, In, Zn, Ba, Ra, Ge, Al, V, Ti, and Sn; lithium titanium oxide (LTO); and lithium vanadium oxide.

More specifically, the (semi)metal-based active material may include a silicon-based active material.

The silicon-based active material may include a compound represented by $SiO_x$ ($0 \leq x < 2$). Since $SiO_2$ does not react with lithium ions and thus cannot store lithium, x is set within the above range, and the silicon-based active material may be SiO.

The average particle diameter ($D_{50}$) of the silicon-based active material may be 1 μm to 30 μm, specifically, 2 μm to 15 μm, in terms of ensuring structural stability during charge and discharge and reducing side reactions with the electrolyte.

The negative electrode may include a negative electrode current collector, and a negative electrode active material layer disposed on at least one surface of the negative electrode current collector. The negative electrode active material may be included in the negative electrode active material layer.

The negative electrode current collector is not particularly limited as long as it has sufficiently high conductivity without causing chemical changes in the battery. Specifically,the negative electrode current collector include copper, stainless steel, aluminum, nickel, titanium, calcined carbon, copper or stainless steel surface-treated with carbon, nickel, titanium, silver, or the like, and an aluminum-cadmium alloy.

The thickness of the negative electrode current collector may have a thickness of 3 μm to 500 μm.

The negative electrode current collector may have fine unevenness formed on the surface to enhance the bonding strength of the negative electrode active material. For example, the negative electrode current collector may be used in various forms, such as a film, a sheet, a foil, a net, a porous body, a foam, and a non-woven fabric.

The negative electrode active material layer may be arranged on at least one side of the negative electrode current collector, specifically, on one or both sides of the negative electrode current collector.

The negative electrode active material may be included in the negative electrode active material layer in an amount of 60 wt% to 99 wt%, specifically,75 wt% to 95 wt%.

Other positive electrode active materials have been described above and will not be discussed.

The negative electrode active material layer may further include a binder and/or a conductive material together with the negative electrode active material.

The binder is used to improve the performance of the battery by improving the adhesion between the negative electrode active material layer and the negative electrode current collector, and may include, for example, at least one selected from the group consisting of polyvinylidene fluoride-hexafluoropropylene copolymer (PVDF-co-HFP), polyvinylidene fluoride (PVDF), polyacrylonitrile, poly(methyl methacrylate), polyvinyl alcohol, carboxymethyl cellulose (CMC), starch, hydroxypropyl cellulose, regenerated cellulose, polyvinyl pyrrolidone, polytetrafluoroethylene, polyethylene, polypropylene, polyacrylic acid, ethylene-propylene-diene monomer (EPDM), sulfonated EPDM, styrene butadiene

rubber (SBR), fluororubber, and those thereof in which hydrogen is substituted with Li, Na, or Ca, and also may include various copolymers thereof.

The binder may be included in the negative electrode active material in an amount of 0.5 wt% to 10 wt%, specifically, 1 wt% to 5 wt%.

The conductive material is not particularly limited as long as it is conductive and does not cause a chemical change in the battery, and examples thereof include graphites such as natural graphite or artificial graphite; carbon blacks such as carbon black, acetylene black, ketjenblack, channel black, furnace black, lamp black, and thermal black; conductive fibers such as carbon fibers or metal fibers; conductive tubes such as carbon nanotubes; fluorocarbon; metal powder such as aluminum or nickel powder; conductive whiskers such as zinc oxide or potassium titanate; conductive metal oxides such as titanium oxide; and conductive materials such as polyphenylene derivatives.

The conductive material may be included in the negative electrode active material in an amount of 0.5 wt% to 10 wt%, specifically, 1 wt% to 5 wt%.

The thickness of the negative electrode active material layer may be 10 μm to 200 μm, specifically, 20 μm to 150 μm.

The negative electrode may be manufactured by coating a negative electrode slurry including a negative electrode active material, a binder, a conductive material, and/or a solvent for forming the negative electrode slurry, on at least one surface of the negative electrode current collector, followed by drying and rolling.

The solvent for forming the negative electrode slurry may include, for example, at least one selected from the group consisting of distilled water, NMP (N-methyl-2-pyrrolidone), ethanol, methanol, and isopropyl alcohol, specifically, may include distilled water, in terms of facilitating dispersion of the negative electrode active material, the binder, and/or the conductive agent. Specifically, the solid content of the negative electrode slurry may be 30 wt% to 80 wt%, specifically, may be about 40 wt% to 70 wt%.

**(3) Separator**

The separator may be interposed between the positive electrode and the negative electrode.

In addition, the separator includes a porous polymer film commonly used as a conventional separator, for example, a porous polymer film made of a polyolefin polymer such as an ethylene homopolymer, a propylene homopolymer, an ethylene/butene copolymer, an ethylene/hexene copolymer, and an ethylene/methacrylate copolymer, which may be used alone or in a laminated manner, or a conventional porous nonwoven fabric, for example, a nonwoven fabric made of high-melting-point glass fiber or polyethylene terephthalate fiber, but is not limited thereto. Further, a coated separator containing ceramic components or polymer materials may be used to ensure heat resistance or mechanical strength, and may optionally be used in a single-layer or multilayer structure.

The shape of the lithium secondary battery of the present invention is not particularly limited, but may be cylindrical, square, pouch, or coin-shaped, using a can.

Hereinafter, the present invention is described through specific examples. However, the following examples are intended to illustrate the invention and are not intended to limit the scope of the present invention. It will be obvious to those skilled in the art that various changes and modifications may be made within the scope of the present description and the technical idea, and that such changes and modifications fall within the scope of the appended claims.

**Examples and Comparative Examples**

Example 1

(Preparation of Non-aqueous Electrolyte)

A mixture of ethylene carbonate (EC) and ethyl methyl carbonate (EMC) in a volume ratio of 20:80 was used as an organic solvent.

$LiPF_6$ as a lithium salt, a compound represented by Formula 4-1 as an additive, and Compound A were added to the organic solvent to prepare a non-aqueous electrolyte.

The $LiPF_6$ was included in the non-aqueous electrolyte at a molar concentration of 1.2 M. The compound represented by Formula 4-1 was included in the non-aqueous electrolyte in an amount of 1 wt%. Compound A was included in the non-aqueous electrolyte in an amount of 1 wt%.

The non-aqueous electrolyte was aged at room temperature for 72 hours, and the compound represented by Formula 4-1 and Compound A were reacted with each other. As a result of analyzing the components in the non-aqueous electrolyte aged for 72 hours through 1H-NMR and LC-MS, the compound represented by Formula 1-a and the compound represented by Formula 1-b were formed in the non-aqueous electrolyte in an amount of 0.5 wt%, the compound represented by Formula 4-1 remained in the non-aqueous electrolyte in an amount of 0.75 wt%, and Compound A

remained in the non-aqueous electrolyte in an amount of 0.7 wt%.

(Preparation of Lithium Secondary Battery)

A positive electrode active material ($Li[Ni_{0.6}Co_{0.1}Mn_{0.3}]O_2$), a conductive material (carbon nanotube), and a binder (PVDF) were added to a solvent, N-methyl-2-pyrrolidone (NMP) in a weight ratio of 97.74:0.70:1.56 to prepare a positive electrode mixture slurry (solid content: 75.5 wt%). The positive electrode mixture slurry was applied to one surface of a positive electrode current collector (Al thin film) having a thickness of 12 μm, and drying and roll pressing were performed to form a positive electrode active material layer (thickness: 136.6 μm), which was used as a positive electrode.

A negative active material (natural graphite), a conductive agent (carbon black), and a binder (SBR-CMC) were added to distilled water as a solvent in a weight ratio of 96.15:1.55:2.30 to prepare a negative electrode mixture slurry (solid content 26 wt%). The negative electrode mixture slurry was applied to one side of a negative electrode current collector (Cu thin film) having a thickness of 8 μm, dried, and roll pressed to prepare a negative electrode.

A polyethylene porous film separator was interposed between the positive and negative electrodes prepared above in a dry room, and then the non-aqueous electrolyte prepared above was injected to manufacture a secondary battery.

Example 2

A non-aqueous electrolyte and a lithium secondary battery were prepared in the same manner as in Example 1, except that Compound A was added to the non-aqueous electrolyte in an amount of 5 wt% instead of 1 wt%.

After aging the non-aqueous electrolyte at room temperature for 72 hours, the compound represented by Formula 4-1 and Compound A reacted with each other to form 0.5 wt% of the compound represented by Formula 1-a and the compound represented by Formula 1-b. The compound represented by Formula 4-1 remained in an amount of 0.75 wt%, and Compound A remained in an amount of 4.75 wt%.

Example 3

A non-aqueous electrolyte and a lithium secondary battery were prepared in the same manner as in Example 1, except that Compound A was added to the non-aqueous electrolyte in an amount of 0.5 wt% instead of 1 wt%.

After aging the non-aqueous electrolyte at room temperature for 72 hours, the compound represented by Formula 4-1 and Compound A reacted with each other to form 0.2 wt% of the compound represented by Formula 1-a and the compound represented by Formula 1-b. The compound represented by Formula 4-1 remained in an amount of 0.9 wt%, and Compound A remained in an amount of 0.4 wt%.

Example 4

A non-aqueous electrolyte and a lithium secondary battery were prepared in the same manner as in Example 1, except that the compound represented by Formula 4-1 was added to the non-aqueous electrolyte in an amount of 5 wt% instead of 1 wt%.

After aging the non-aqueous electrolyte at room temperature for 72 hours, the compound represented by Formula 4-1 and Compound A reacted with each other to form 0.5 wt% of the compound represented by Formula 1-a and the compound represented by Formula 1-b. The compound represented by Formula 4-1 remained in an amount of 4.75 wt%, and Compound A remained in an amount of 0.75 wt%.

Example 5

A non-aqueous electrolyte and a lithium secondary battery were prepared in the same manner as in Example 1, except that the compound represented by Formula 4-1 was added to the non-aqueous electrolyte in an amount of 0.5 wt% instead of 1 wt%.

After aging the non-aqueous electrolyte at room temperature for 72 hours, the compound represented by Formula 4-1 and Compound A reacted with each other to form 0.2 wt% of the compound represented by Formula 1-a and the compound represented by Formula 1-b. The compound represented by Formula 4-1 remained in an amount of 0.4 wt%, and Compound A remained in an amount of 0.9 wt%.

Comparative Example 1

A non-aqueous electrolyte and a lithium secondary battery were prepared in the same manner as in Example 1,

except that the compound represented by Formula 4-1 and Compound A were not added to the non-aqueous electrolyte.

Comparative Example 2

A non-aqueous electrolyte and a lithium secondary battery were prepared in the same manner as in Example 1, except that the compound represented by Formula 4-1 was not added in the non-aqueous electrolyte.

Comparative Example 3

A non-aqueous electrolyte and a lithium secondary battery were prepared in the same manner as in Example 1, except that Compound A was not added to the non-aqueous electrolyte.

Comparative Example 4

A non-aqueous electrolyte and a lithium secondary battery were prepared in the same manner as in Example 1, except that 1 wt% of 1,3-propane sultone (PS) was added to the non-aqueous electrolyte, instead of 1 wt% of Compound A.

Experimental Example

Experimental Example 1: Evaluation of high temperature cycle performance

The lithium secondary batteries manufactured in Examples 1 to 5 and Comparative Examples 1 to 4 were subjected to 200 cycles, each of which includes charging to 4.4 V, 0.05 C at 45 °C under conditions of CC/CV, 0.33 C, and discharging to 2.5 V under conditions of CC, 0.33 C using an electrochemical charger/discharger.

(1) Capacity Retention Rate

The capacity retention rate was calculated using the equation below, and the results are indicated in Table 1 below.

Capacity retention rate (%)={(discharge capacity after 200 cycles/discharge capacity after 1 cycle)} ×100

(2) Resistance Increase Rate

After one cycle of charge and discharge, the discharge capacity after one cycle was measured using the electrochemical charger/discharger, the SOC was adjusted to SOC 50%, and then, a pulse of 2.5 C was applied for 10 seconds to calculate the initial resistance from the difference between the voltage before the pulse application and the voltage after the pulse application.

After 200 cycles of charge and discharge, the resistance after 200 cycles was calculated using the same method as described above, and the resistance increase rate was calculated using the equation below. The results are indicated in Table 1 below.

Resistance increase rate (%) = (resistance after 200 cycles - initial resistance)/initial resistance × 100

[Table 1]

| | Capacity retention rate (%) | Resistance increase rate (%) |
|---|---|---|
| Example 1 | 95 | 8 |
| Example 2 | 94 | 10 |
| Example 3 | 95 | 11 |
| Example 4 | 96 | 12 |
| Example 5 | 95 | 10 |
| Comparative Example 1 | 72 | 36 |
| Comparative Example 2 | 76 | 30 |

(continued)

| | Capacity retention rate (%) | Resistance increase rate (%) |
|---|---|---|
| Comparative Example 3 | 77 | 28 |
| Comparative Example 4 | 80 | 25 |

Referring to Table 1, it can be confirmed that the lithium secondary batteries of Examples 1 to 5 using non-aqueous electrolytes including the compound represented by Formula 1 exhibit superior high-temperature cycle performance, compared to Comparative Examples.

Experimental Example 2: Evaluation of high temperature storage performance

The lithium secondary batteries manufactured in Examples 1 to 5 and Comparative Examples 1 to 4 were charged to 4.4 V, 0.05 C at 25°C under conditions of CC/CV, 0.33 C and discharged to 2.5 V under conditions of CC, 0.33 C to perform initial charge/discharge, and then charged to 4.4 V, 0.05 C at 25°C under conditions of CC/CV, 0.33 C and stored at 60°C for 8 weeks.

(1) Capacity Retention Rate

After 8 weeks of storage, the lithium secondary battery was charged to 4.4 V, 0.05 C at 25°C under conditions of CC/CV, 0.33 C and discharged to 2.5 V under conditions of CC, 0.33 C to measure the capacity during discharge.

The capacity retention rate was evaluated according to the following equation, and the results are indicated in Table 2 below.

Capacity retention rate (%)={(discharge capacity after 8 weeks of storage/initial discharge capacity)} × 100

(2) Resistance Increase Rate

After the initial charge and discharge as described above, the capacity was checked at room temperature, then the battery was charged to SOC 50% based on the discharge capacity, and discharged for 10 seconds with a current of 2.5 C. Then, the resistance was measured from the voltage drop difference at that time, which was used as the initial resistance, and after 8 weeks of storage at 60°C, the resistance was measured using the same method, which was used as the final resistance. Then, the resistance increase rate was calculated using the following equation. The results are indicated in Table 2 below.

Resistance increase rate (%)=(final resistance-initial resistance)/initial resistance × 100

[Table 2]

| | Capacity retention rate (%) | Resistance increase rate (%) |
|---|---|---|
| Example 1 | 94 | 9 |
| Example 2 | 91 | 9 |
| Example 3 | 93 | 11 |
| Example 4 | 91 | 10 |
| Example 5 | 92 | 10 |
| Comparative Example 1 | 70 | 32 |
| Comparative Example 2 | 73 | 24 |
| Comparative Example 3 | 72 | 25 |
| Comparative Example 4 | 79 | 20 |

Referring to Table 2, it can be confirmed that the lithium secondary batteries of Examples 1 to 5 using non-aqueous electrolytes including the compound represented by Formula 1 exhibit superior high-temperature storage

performance compared to Comparative Examples.

## Claims

**1.** A non-aqueous electrolyte comprising:

a lithium salt;
an organic solvent; and
an additive,
wherein the additive contains a compound represented by Formula 1:

(Formula 1)

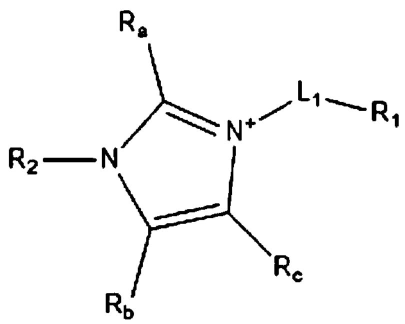

wherein,
$R_1$ is a substituent represented by Formula 2 below,
$L_1$ is a direct bond or a substituted or unsubstituted alkylene group having 1 to 6 carbon atoms,
$R_2$ is hydrogen or a substituent represented by Formula 3 below, and
$R_a$, $R_b$, and $R_c$ are each independently hydrogen, an alkyl group having 1 to 3 carbon atoms, or -CN,

(Formula 2)

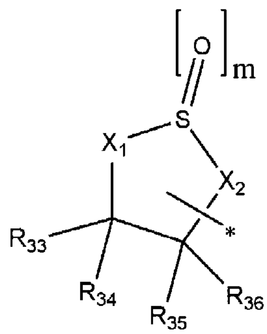

wherein,
m is 1 or 2,
$X_1$ and $X_2$ are each independently -O- or $-C(R_{31})(R_{32})-$, provided that at least one of $X_1$ and $X_2$ is -O-,
$R_{31}$ to $R_{36}$ are each independently hydrogen, an alkyl group having 1 to 6 carbon atoms, $-C(=O)-R_4$, or $-R_5-O-C(=O)-R_6$,
$R_4$ and $R_6$ are each independently a substituted or unsubstituted alkyl group having 1 to 6 carbon atoms, an alkenyl group having 2 to 20 carbon atoms, an alkynyl group having 2 to 20 carbon atoms, or a substituted or unsubstituted aryl group having 6 to 20 carbon atoms,
$R_5$ is a substituted or unsubstituted alkylene group having 1 to 6 carbon atoms, each of substituents of $L_1$, $R_4$, $R_5$, and $R_6$ are each independently at least one selected from the group consisting of deuterium, -F, -Cl, -Br, -I, -CN, $-NO_2$, and $-SO_3$, and

* is a bonding site located at one of $R_{31}$, $R_{32}$, $R_{33}$, $R_{34}$, $R_{35}$, and $R_{36}$,

(Formula 3)

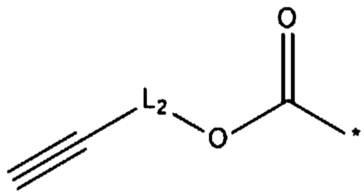

wherein,
* is a bonding site, and
$L_2$ is an alkylene group having 1 to 3 carbon atoms.

**2.** The non-aqueous electrolyte according to claim 1, wherein the compound represented by Formula 1 includes at least one of compounds represented by Formulas 1-1 and 1-2:

(Formula 1-1)

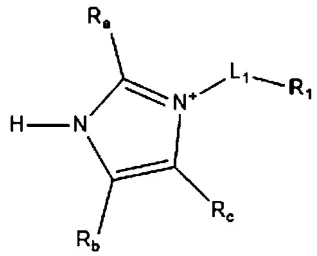

(Formula 1-2)

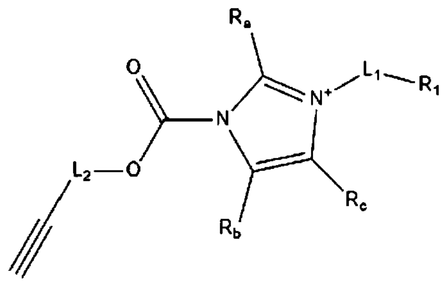

wherein $R_a$, $R_b$, $R_c$, $R_1$, $L_1$, and $L_2$ are as defined in Formula 1.

**3.** The non-aqueous electrolyte according to claim 1, wherein $L_1$ is a methylene group.

**4.** The non-aqueous electrolyte according to claim 1, wherein the substituent represented by Formula 2 is any one selected from substituents CS-1 to CS-15:

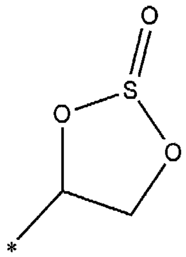
CS-1

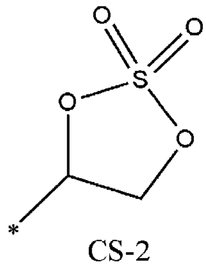
CS-2

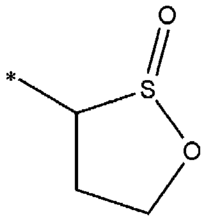
CS-3

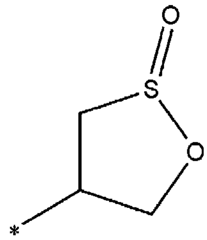
CS-4

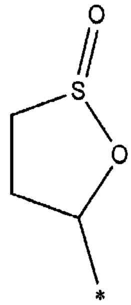
CS-5

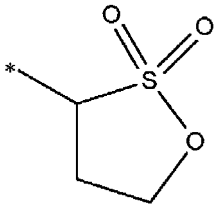
CS-6

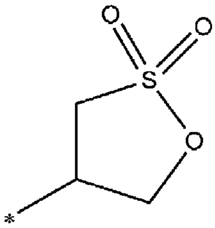
CS-7

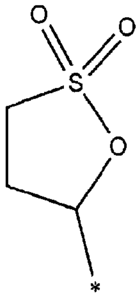
CS-8

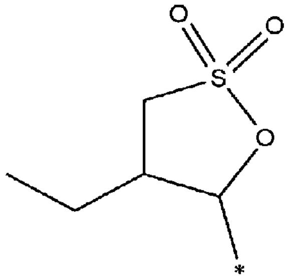
CS-9

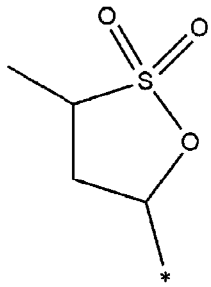
CS-10

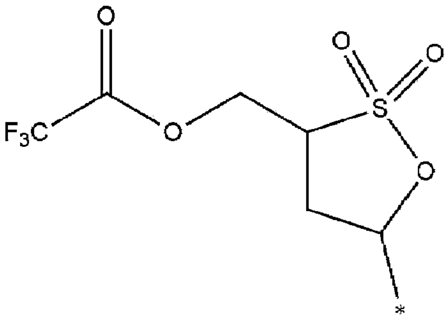
CS-11

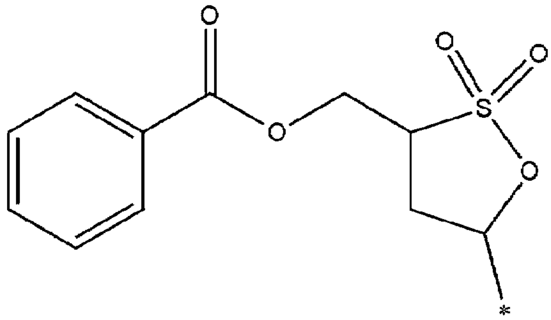
CS-12

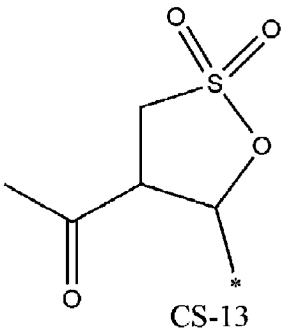
CS-13

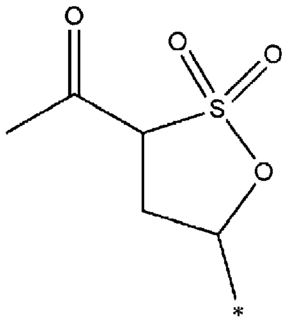
CS-14

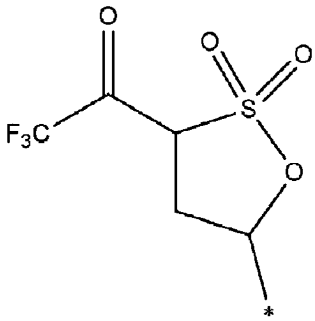
CS-15

**5.** The non-aqueous electrolyte according to claim 4, wherein the substituent represented by Formula 2 is one selected from the group consisting of CS-1, CS-2, CS-5, CS-8, CS-10, and CS-11.

**6.** The non-aqueous electrolyte according to claim 1, wherein $L_2$ is a methylene group.

**7.** The non-aqueous electrolyte according to claim 1, wherein the compound represented by Formula 1 includes at least one of compounds represented by Formulas 1-A and 1-B:

(Formula 1-A)

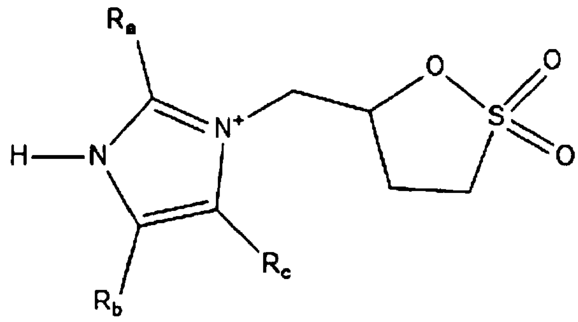

(Formula 1-B)

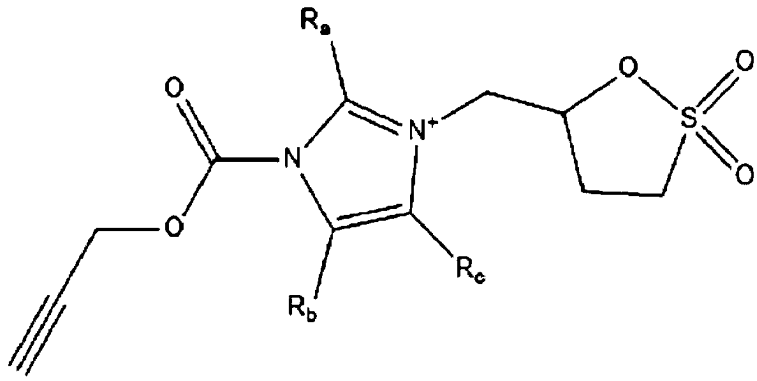

wherein $R_a$, $R_b$, and $R_c$ are as defined in Formula 1.

**8.** The non-aqueous electrolyte according to claim 1, wherein the compound represented by Formula 1 includes at least one of compounds represented by Formulas 1-a and 1-b:

(Formula 1-a)

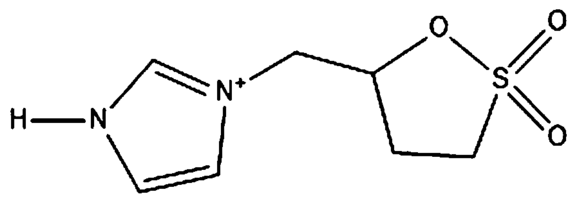

(Formula 1-b)

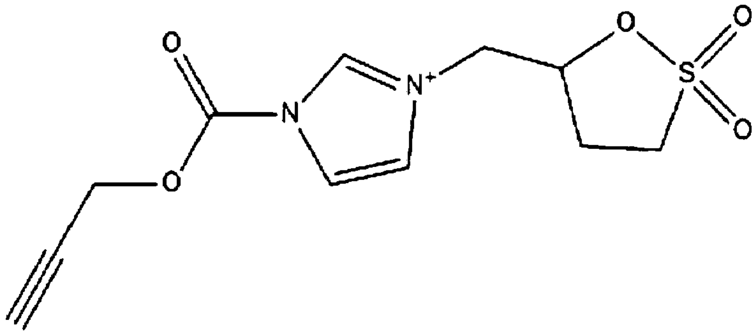

**9.** The non-aqueous electrolyte according to claim 1, wherein the additive further includes a compound represented by Formula 4:

(Formula 4)

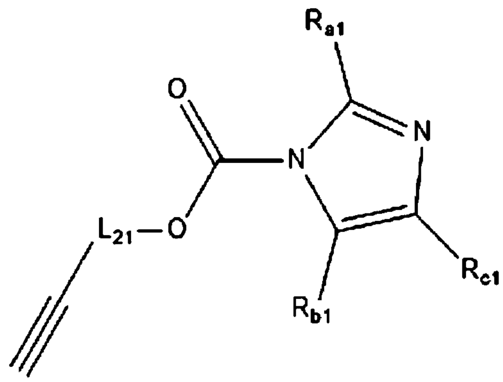

wherein,

$R_{a1}$, $R_{b1}$ and $R_{c1}$ are each independently hydrogen, an alkyl group having 1 to 3 carbon atoms, or -CN, and $L_{21}$ is an alkylene group having 1 to 3 carbon atoms.

**10.** The non-aqueous electrolyte according to claim 1, wherein the additive further includes a compound represented by Formula 5:

(Formula 5)

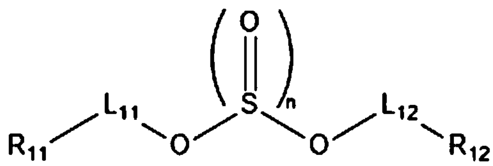

wherein,

n is 1 or 2,
$L_{11}$ and $L_{12}$ are each independently a direct bond or a substituted or unsubstituted alkylene group having 1 to 6 carbon atoms, and
$R_{11}$ and $R_{12}$ are each independently a substituent represented by Formula 6:

(Formula 6)

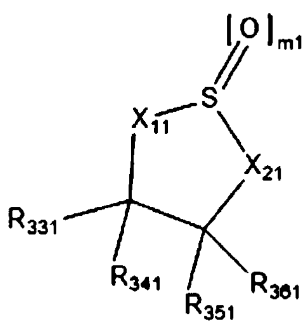

wherein,
m1 is 1 or 2,

$X_{11}$ and $X_{21}$ are each independently -O- or -C($R_{311}$)($R_{321}$)-, provided that at least one of $X_{11}$ and $X_{21}$ is -O-,
$R_{311}$, $R_{321}$, $R_{331}$, $R_{341}$, $R_{351}$, and $R_{361}$ are each independently hydrogen, an alkyl group having 1 to 6 carbon atoms, -C(=O)-$R_{41}$, or -$R_{51}$-O-C(=O)-$R_{61}$,
$R_{41}$ and $R_{61}$ are each independently a substituted or unsubstituted alkyl group having 1 to 6 carbon atoms, an alkenyl group having 2 to 20 carbon atoms, an alkynyl group having 2 to 20 carbon atoms, or substituted or unsubstituted aryl group having 6 to 20 carbon atoms,
$R_{51}$ is a substituted or unsubstituted alkylene group having 1 to 6 carbon atoms,
each of substituents of $L_{11}$, $L_{21}$, $R_{41}$, $R_{51}$, and $R_{61}$ are each independently at least one selected from the group consisting of deuterium, -F, -Cl, -Br, -I, -CN, -$NO_2$, and - $SO_3$,
* is a bonding site located at one of $R_{311}$, $R_{321}$, $R_{331}$, $R_{341}$, $R_{351}$, and $R_{361}$,
when $L_{11}$ and $L_{21}$ are both direct bonds, $R_{11}$ and $R_{21}$ are not CS1-7 below at the same time, and
when $L_{11}$ and $L_{21}$ are both methylene groups and n is 2, $R_{11}$ and $R_{21}$ are not CS1-2 below at the same time:

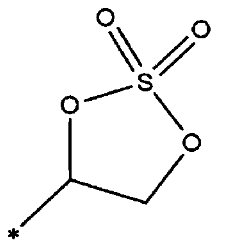
CS1-2

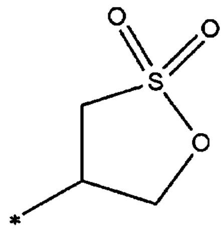
CS1-7

**11.** The non-aqueous electrolyte according to claim 10, wherein the substituent represented by Formula 6 is any one selected from the group consisting of CS1-1 to CS1-15:

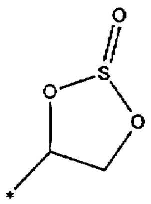
CS1-1

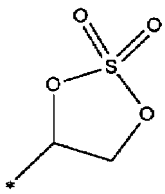
CS1-2

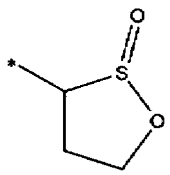
CS1-3

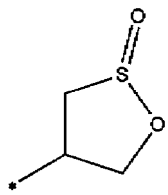
CS1-4

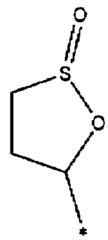
CS1-5

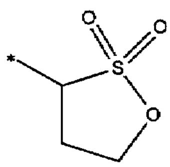
CS1-6

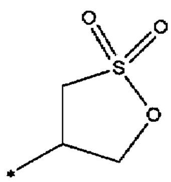
CS1-7

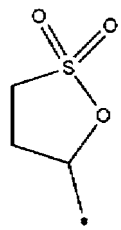
CS1-8

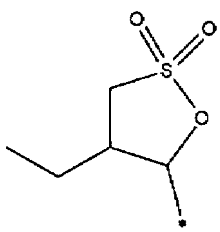
CS1-9

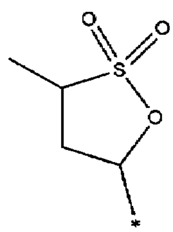
CS1-10

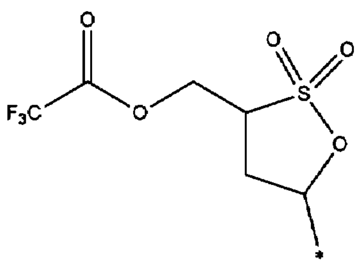
CS1-11

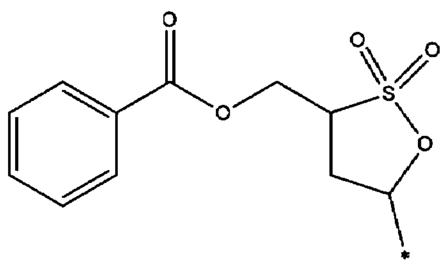
CS1-12

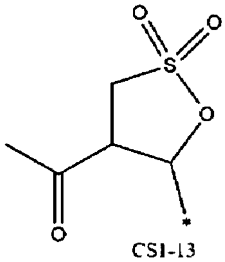
CS1-13

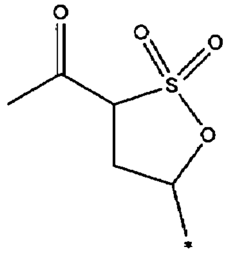
CS1-14

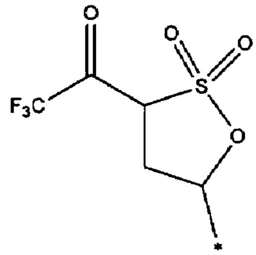
CS1-15

**12.** The non-aqueous electrolyte according to claim 11, wherein the compound represented by Formula 5 includes at least one compound selected from the group consisting of compounds A to Q:

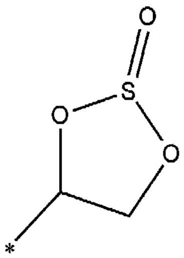
CS-1

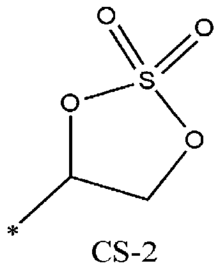
CS-2

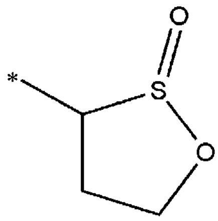
CS-3

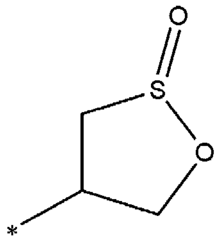
CS-4

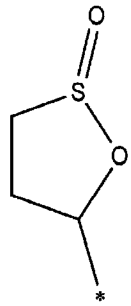
CS-5

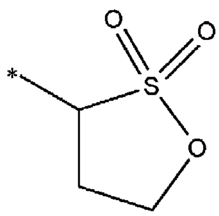
CS-6

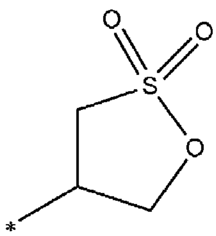
CS-7

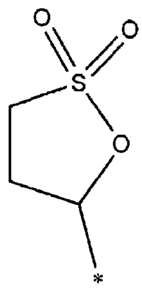
CS-8

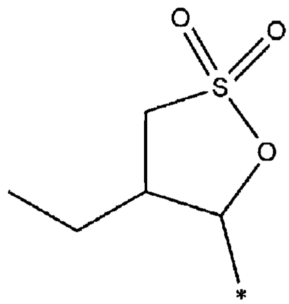
CS-9

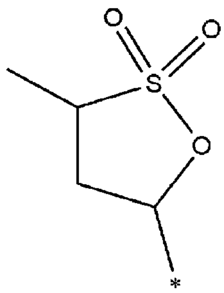
CS-10

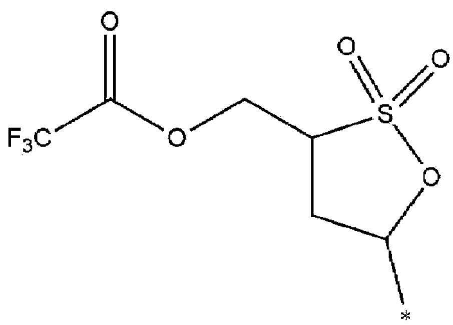
CS-11

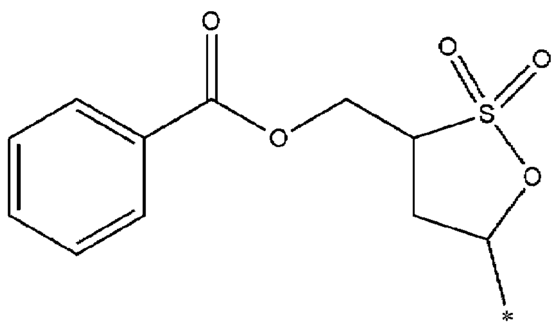
CS-12

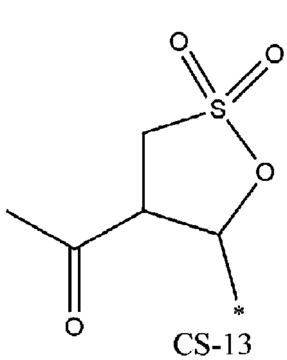
CS-13

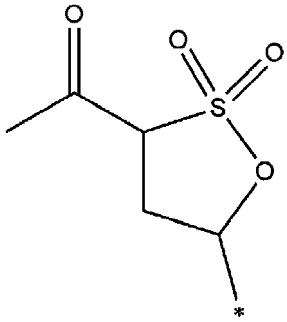
CS-14

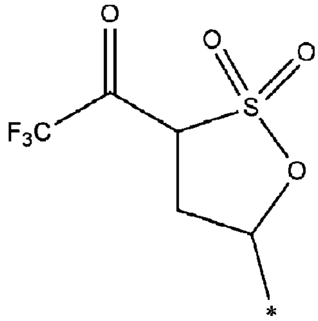
CS-15 .

**13.** The non-aqueous electrolyte according to claim 1, wherein the compound represented by Formula 1 is included in an amount of 0.01 wt% to 10 wt% based on the total weight of the non-aqueous electrolyte.

**14.** The non-aqueous electrolyte according to claim 1, wherein the lithium salt incudes at least one selected from the group consisting of LiCl, LiBr, LiI, $LiBF_4$, $LiClO_4$, $LiAlO_4$, $LiAlCl_4$, $LiPF_6$, $LiSbF_6$, $LiAsF_6$, $LiB_{10}Cl_{10}$, LiBOB ($LiB(C_2O_4)_2$), $LiCF_3SO_3$, LiFSI ($LiN(SO_2F)_2$), $LiCH_3SO_3$, $LiCF_3CO_2$, $LiCH_3CO_2$, and LiBETI ($LiN(SO_2CF_2CF_3)_2$).

**15.** A lithium secondary battery comprising:

a positive electrode;
a negative electrode disposed in an opposite side to the positive electrode;
a separator interposed between the positive electrode and the negative electrode; and
the non-aqueous electrolyte according to claim 1.

**16.** The lithium secondary battery according to claim 15, wherein the positive electrode includes a positive electrode active material, and

the positive electrode active material includes a lithium transition metal oxide represented by Formula P-1:

(Formula P-1) $Li_{1+x}[Ni_aCo_bMn_cM^1_d]O_{2+w}$

wherein,
x, a, b, c, d, and w satisfy $0 \le x \le 0.5$, $a+b+c+d = 1$, $0.5 \le a \le 0.7$, $0 \le b \le 0.15$, $c=1-a-b-d$, $0 \le d \le 0.1$, $0 \le b/a \le 0.2$, $1 \le a/c \le 3$, $0 \le w \le 1$, respectively, and
$M^1$ is at least one selected from W, Cu, Fe, V, Cr, Ti, Zr, Zn, Al, In, Ta, Y, La, Sr, Ga, Sc, Gd, Sm, Ca, Ce, Nb, Mg, B, and Mo.

## INTERNATIONAL SEARCH REPORT

International application No.
**PCT/KR2024/020701**

**A. CLASSIFICATION OF SUBJECT MATTER**

**H01M 10/0567**(2010.01)i; **H01M 10/0525**(2010.01)i; **H01M 4/525**(2010.01)i; **H01M 4/505**(2010.01)i

According to International Patent Classification (IPC) or to both national classification and IPC

**B. FIELDS SEARCHED**

Minimum documentation searched (classification system followed by classification symbols)

H01M 10/0567(2010.01); H01M 10/052(2010.01); H01M 10/0525(2010.01); H01M 10/0566(2010.01); H01M 4/58(2010.01); H01M 4/60(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus) & keywords: 비수 전해질(non-aqueous electrolyte), 첨가제(additive), 이미다졸리움(imidazolium), 환형 황산화물(cyclic sulfur oxide), 프로파질기(propargyl group), 리튬 이차전지(lithium secondary battery)

**C. DOCUMENTS CONSIDERED TO BE RELEVANT**

| Category* | Citation of document, with indication, where appropriate, of the relevant passages | Relevant to claim No. |
|---|---|---|
| A | KR 10-2021-0138937 A (LG ENERGY SOLUTION, LTD.) 22 November 2021 (2021-11-22)<br>See claim 1; paragraphs [0083]-[0085]; and example 2. | 1-16 |
| A | US 2006-0210876 A1 (KUBOKI, T. et al.) 21 September 2006 (2006-09-21)<br>See claims 1 and 17-20; and paragraph [0053]. | 1-16 |
| A | KR 10-2022-0135208 A (LG CHEM, LTD. et al.) 06 October 2022 (2022-10-06)<br>See claims 1-11; and paragraphs [0074], [0083] and [0085]. | 1-16 |
| A | KR 10-0879363 B1 (UNIVERSITY-INDUSTRY COOPERATION GROUP OF KYUNG HEE UNIVERSITY) 19 January 2009 (2009-01-19)<br>See claims 1-11. | 1-16 |
| A | KR 10-2240799 B1 (EP CHEMTECH CO., LTD.) 16 April 2021 (2021-04-16)<br>See claims 1-10; and paragraph [0077]. | 1-16 |

☐ Further documents are listed in the continuation of Box C.    ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 March 2025** | **24 March 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**
**Information on patent family members**

International application No.
**PCT/KR2024/020701**

| Patent document cited in search report | Publication date (day/month/year) | Patent family member(s) | Publication date (day/month/year) |
|---|---|---|---|
| KR 10-2021-0138937 A | 22 November 2021 | None | |
| US 2006-0210876 A1 | 21 September 2006 | JP 2006-260952 A | 28 September 2006 |
| | | JP 4202334 B2 | 24 December 2008 |
| | | US 7682736 B2 | 23 March 2010 |
| KR 10-2022-0135208 A | 06 October 2022 | CN 116491005 A | 25 July 2023 |
| | | EP 4213265 A1 | 19 July 2023 |
| | | JP 2023-545066 A | 26 October 2023 |
| | | US 2024-0014443 A1 | 11 January 2024 |
| | | WO 2022-211425 A1 | 06 October 2022 |
| KR 10-0879363 B1 | 19 January 2009 | None | |
| KR 10-2240799 B1 | 16 April 2021 | KR 10-2020-0133450 A | 30 November 2020 |

Form PCT/ISA/210 (patent family annex) (July 2022)